# EUROPEAN PATENT SPECIFICATION

(11) **EP 2 086 968 B1**
(45) Date of publication and mention of the grant of the patent: **08.02.2012**
(21) Application number: 07827038.6
(22) Date of filing: 09.11.2007
(51) Int. Cl.: C07D 413/14, C07D 471/04, A61K 31/4375, A61K 31/4709, A61P 31/00

(54) **5-HYDROXYMETHYL-OXAZOLIDIN-2-ONE DERIVATIVES**
5-HYDROXYMETHYLOXAZOLIDIN-2-ONDERIVATE
DÉRIVÉS DE 5-HYDROXYMÉTHYL-OXAZOLIDIN-2-ONE

(30) Priority: 10.11.2006 WO PCT/IB2006/054189
(43) Date of publication of application: 12.08.2009
(73) Proprietor: Actelion Pharmaceuticals Ltd., 4123 Allschwil (CH)
(72) Inventor: HUBSCHWERLEN, Christian, 68480 Durmenach (FR); PANCHAUD, Philippe, 4123 Allschwil (CH); SPECKLIN, Jean-Luc, 68680 Kembs (FR)
(74) Representative: Ruhlmann, Eric
(86) International application number: PCT/IB2007/054557
(87) International publication number: WO 2008/056335

(56) References cited:
- WO-A-02/059116
- WO-A-2004/096221
- WO-A-2005/058886
- SU-A3- 1 156 597
- US-A- 4 461 773
- ERA-CABRERA, L ET AL: "In vitro activities of DA-7157 and DA-7218 against Mycobacterium tuberculosis and Nocardia brasiliensis" ANTIMICROBIAL AGENTS AND CHEMOTHERAPY, vol. 50, no. 9, September 2006 (2006-09), pages 3170-3172, XP002473516

## Description

The present invention concerns novel chimeric antibiotics that are obtained from oxazolidinone derivatives linked to a quinolone or naphthyridinone via a spacer, pharmaceutical antibacterial compositions containing them and the use of these compounds in the manufacture of a medicament for the treatment of infections (e.g. bacterial infections). These chimeric compounds are useful antimicrobial agents effective against a variety of human and veterinary pathogens including among others Gram-positive aerobic bacteria, Gram-negative bacteria, anaerobic organisms and acid-fast organisms.

The intensive use of antibiotics has exerted a selective evolutionary pressure on microorganisms to produce genetically based resistance mechanisms. Modern medicine and socio-economic behaviour exacerbate the problem of resistance development by creating slow growth situations for pathogenic microbes, e.g. in artificial joints, and by supporting long-term host reservoirs, e.g. in immuno-compromised patients.

In hospital settings, an increasing number of strains of *Staphylococcus areus, Streptococcus pneumonia, Enterococcus spp.,* and *Pseudonionas aeruginosa,* major sources of infections, are becoming multi-drug resistant and therefore difficult if not impossible to treat:
- *S. aureus* is β-lactam, quinolone and now even vancomycin resistant;
- *S. pneumoniae* is becoming resistant to penicillin, quinolone and even to new macrolides;
- *Enteroccocci* are quinolone and vancomycin resistant and β-lactams were never efficacious against these strains.

Further new emerging organisms like *Acinetobacter spp.* or *C*. *difficile,* which have been selected during therapy with the currently used antibiotics, are becoming a real problem in hospital settings.

In addition, microorganisms that are causing persistent infections are increasingly being recognized as causative agents or cofactors of severe chronic diseases like peptic ulcers or heart diseases.

In a chimeric molecule two or more molecules that exist separately in their native state are joined together to form a single entity (i.e. molecule) having the desired functionality of all of its constituent molecules.

Molecules wherein two antibiotics that have two different modes of action have been linked have been reported in the literature (*e.g.* Journal of Antimicrobial Chemotherapy (1994), 33, 197-200). Many of them are however such that the two antibiotic parts are released after biological activation (*e.g*. central ester cleavage, beta-lactam cleavage). Chemically and biochemically stable chimeric molecules that bind, as such, in two different targets have been more seldom reported. For example, oxazolidinone-quinolone hybrids have been reported as useful antimicrobial agents effective against a variety of multi-drug resistant pathogens (WO 03/032962, WO 03/031443 and WO 2004/096221, WO 2005/023801 and WO 2005/058888). Further, synthesis and biological evaluation of these hybrids (Bioorg. & Med. Chem. (2003), 11, 2313-2319) and the influence of the central spacer on the antibacterial activity in the structure-activity relationship in the oxazolidinone-quinolone series have also been reported (Bioorg. Med. Chem. Lett. (2003), 13, 4229-4233). All these derivatives contain a 4-aminomethyl-oxazolidinone rest as part of the oxazolidinone pharmacophore.

It has now been surprisingly found that the chimeric derivatives of formula I as defined hereafter are particularly effective antimicrobial agents that show effective against a variety of multi-drug resistant bacteria.

Thus, the present invention relates to compounds of formula I 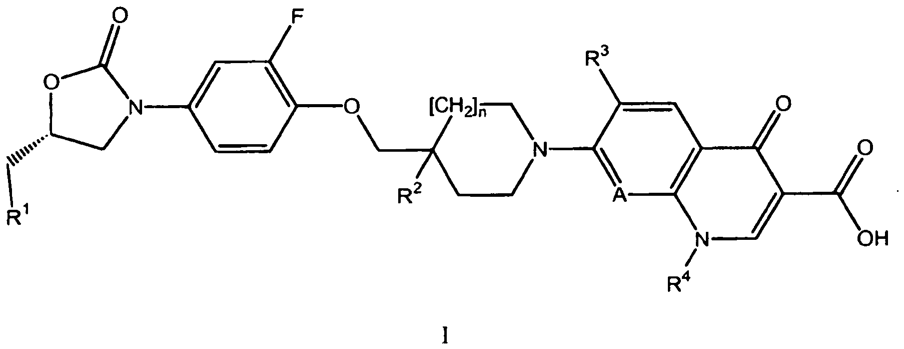 wherein
R¹ represents OH, OPO₃H₂ or OCOR⁵;
R² represents H, OH or OPO₃H₂;
A represents N or CR⁶;
R³ represents H or fluorine;
R⁴ is H, (C₁-C₃) alkyl or cycloalkyl;
R⁵ is the residue of a naturally occurring amino acid, of the enantiomer of a naturally occurring amino acid or of dimethylaminoglycine;
R⁶ represents H, alkoxy or halogen; and
n is 0 or 1;
and to salts (in particular pharmaceutically acceptable salts) of compounds of formula I.

The compounds of formula I may contain one or more stereogenic or asymmetric centers, such as one or more asymmetric carbon atoms. The compounds of formula I may thus be present as mixtures of stereoisomers or preferably as pure stereoisomers. Mixtures of stereoisomers may be separated in a manner known to a person skilled in the art.

The following paragraphs provide definitions of the various chemical moieties for the compounds according to the invention and are intended to apply uniformly throughout the specification and claims unless an otherwise expressly set out definition provides a broader or narrower definition.

Unless specified otherwise, the term "alkyl" (whether used alone or in combination) refers to a saturated straight or branched chain alkyl group containing 1 to 6 carbon atoms, and preferably 1 to 3 carbon atoms. Representative examples of alkyl groups include, but are not limited to, methyl, ethyl, propyl, *iso*-propyl, butyl, *iso*-butyl, *sec*-butyl, *tert-*butyl, *n*-pentyl, neopentyl, *iso*-pentyl, *n*-hexyl and *iso*-hexyl. The term "(C₁-Cₓ)alkyl" (x being an integer) refers to a saturated straight or branched chain alkyl group containing 1 to x carbon atoms.

The term "alkoxy" refers to a saturated straight or branched chain alkoxy group, containing 1 to 6 carbon atoms, and preferably 1 to 3 carbon atoms. Representative examples of alkoxy groups include, but are not limited to, methoxy, ethoxy, propoxy, *iso*-propoxy, *n*-butoxy, *iso*-butoxy, *sec*-butoxy, *tert*-butoxy or *n*-hexyloxy. The term "(C₁-Cₓ)alkoxy" (x being an integer) refers to a straight or branched chain alkoxy group containing 1 to x carbon atoms.

The term "halogen" refers to fluorine, chlorine, bromine or iodine, and preferably to fluorine or chlorine.

The term "cycloalkyl", used alone or in combination, refers to a saturated cyclic hydrocarbon moiety containing 3 to 6 carbon atoms and preferably 3 to 5 carbon atoms. Representative examples of cycloalkyl groups include, but are not limited to, cyclopropyl and cyclopentyl.

When it is written that R⁵ is the residue of an amino acid, it is meant thereby that R⁵-COOH is the corresponding amino acid.

The term "pharmaceutically acceptable salts" refers to non-toxic, inorganic or organic acid and/or base addition salts. Reference can be made to "Salt selection for basic drugs", Int. J. Pharm. (1986), 33, 201-217.

Unless used regarding temperatures, the term "about" placed before a numerical value "X" refers in the current application to an interval extending from X minus 10% of X to X plus 10% of X, and preferably to an interval extending from X minus 5% of X to X plus 5% of X. In the particular case of temperatures, the term "about" placed before a temperature "Y" refers in the current application to an interval extending from the temperature Y minus 10°C to Y plus 10°C, and preferably to an interval extending from Y minus 5°C to Y plus 5°C; besides, room temperature shall mean in the current patent application 25°C.

In particular, the invention relates to compounds of formula I that are also compounds of formula I_{CE} 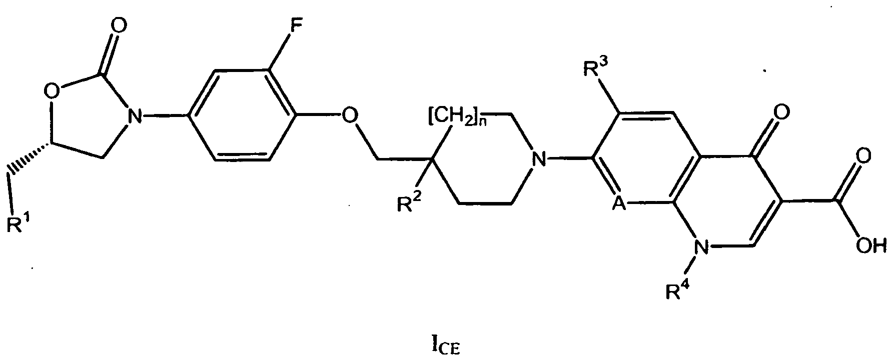 wherein
R¹ represents OH, OPO₃H₂ or OCOR⁵;
R² represents H, OH or OPO₃H₂;
A represents N or CR⁶;
R³ represents fluorine;
R⁴ represents H, (C₁-C₃) alkyl or cycloalkyl;
R⁵ is the residue of a naturally occurring amino acid (in particular the residue of Ala);
R⁶ represents H or alkoxy; and
n is 0 or 1;
and to salts (in particular pharmaceutically acceptable salts) of compounds of formula I_{CE}.

According to a first main embodiment of this invention, the compounds of formula I are such that n is 0. Such compounds will be hereafter referred to as "compounds of formula I₅".

According to one particular variant of the first main embodiment, the compounds of formula I₅ will be such that they have the following stereochemistry:

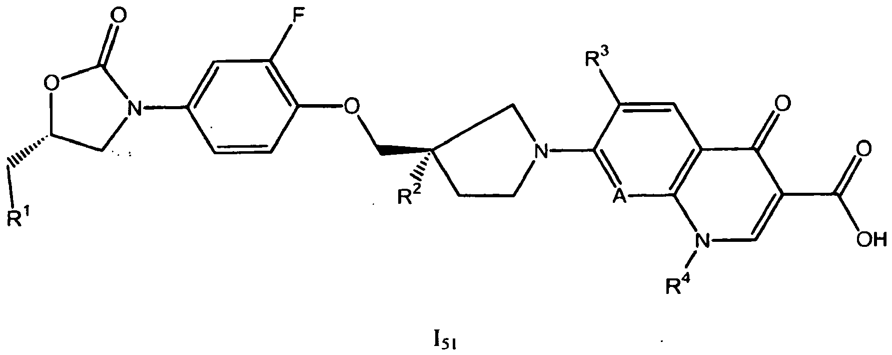

According to another variant of the first main embodiment, the compounds of formula I₅ will be such that they have the following stereochemistry:

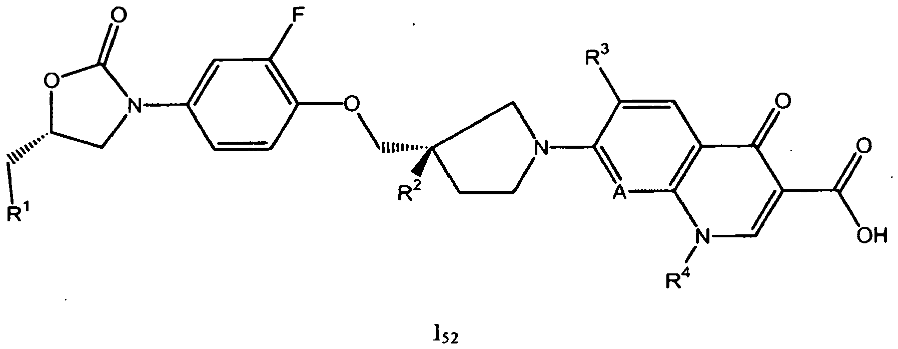

According to a second main embodiment of this invention, the compounds of formula I are such that n is 1. Such compounds will be hereafter referred to as "compounds of formula I₆".

According to a further main embodiment of this invention, the compounds of formula I will be such that they are also compounds of formula I_{D} 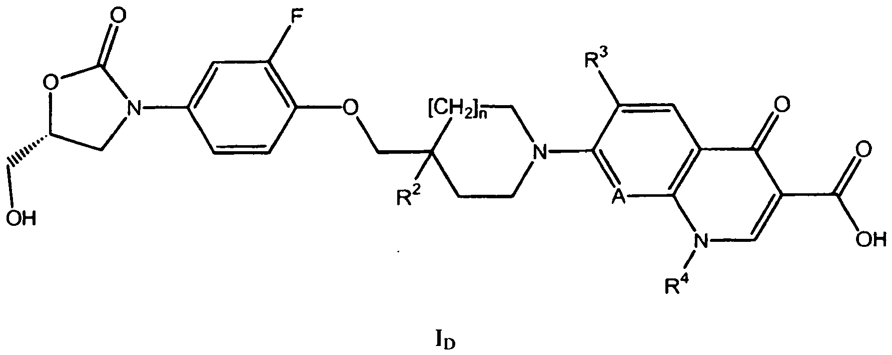 wherein
R² represents H or OH;
A represents N or CR⁶;
R³ represents fluorine;
R⁴ represents H, (C₁-C₃) alkyl or cycloalkyl;
R⁶ represents H or alkoxy; and
n is 0 or 1.

According to yet another main embodiment of this invention, the compounds of formula I will be such that they are also compounds of formula I_{PDG} 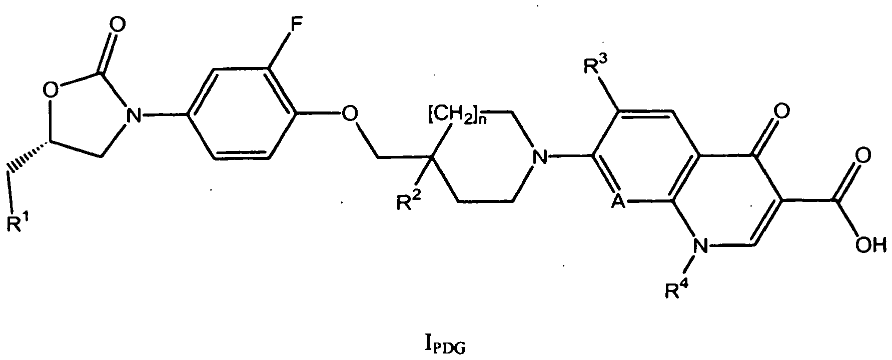 wherein
R¹ represents OH and R² represents OPO₃H₂, or R¹ represents OPO₃H₂ or OCOR⁵ and R² represents H, OH or OPO₃H₂;
A represents N or CR⁶;
R³ represents fluorine;
R⁴ represents H, (C₁-C₃) alkyl or cycloalkyl;
R⁵ is the residue of a naturally occurring amino acid (in particular the residue of Ala);
R⁶ represents H or alkoxy; and
n is 0 or 1.

According to a particular embodiment of this invention, the compounds of formula I will be such that A represents N.

According to another particular embodiment of this invention, the compounds of formula I will be such that A represents CR⁶. In such case, R⁶ will preferably represent H or alkoxy (and in particular H or methoxy).

According to an important variant of this invention, the compounds of formula I will be such that R¹ is OH.

According to another important variant of this invention, the compounds of formula I will be such that R¹ is OPO₃H₂ or OCOR⁵.

According to yet another important variant of this invention, the compounds of formula I will be such that R² is H.

According to yet another important variant of this invention, the compounds of formula I will be such that R² is OH.

According to a further important variant of this invention, the compounds of formula I will be such that R² is OPO₃H₂.

Preferably, the amino acid residue R⁵ is such that R⁵-COOH represents a natural amino acid (notably Ala).

Preferred compounds of formula I are also those wherein at least one of the following characteristics is present:
❖ A represents CR⁶;
❖ R¹ represents OH or OPO₃H₂;
❖ R² represents H or OH;
❖ R³ represents fluorine;
❖ R⁴ represents (C₁-C₃)alkyl or cycloalkyl.

More preferred compounds of formula I are those wherein at least one of the following characteristics is present:
❖ n is 0;
❖ A represents CR⁶, R⁶ representing H or alkoxy (and preferably H or methoxy);
❖ R¹ represents OH;
❖ R² represents H or OH;
❖ R³ represents fluorine;
❖ R⁴ represents cycloalkyl.

Even more preferred compounds of formula I are those wherein at least one of the following characteristics is present:
❖ n is 0;
❖ A represents CR⁶, R⁶ representing H or methoxy;
❖ R¹ represents OH;
❖ R² represents H or OH (and notably OH);
❖ R³ represents fluorine;
❖ R⁴ represents (C₃-C₅)cycloalkyl (and in particular cyclopropyl).

The following compounds of formula I are particularly preferred:
- 1-cyclopropyl-6-fluoro-7-{4-[2-fluoro-4-((*R*)-5-hydroxymethyl-2-oxo-oxazolidin-3-yl)-phenoxymethyl]-4-hydroxy-piperidin-1-yl}-4-oxo-1,4-dihydro-quinoline-3-carboxylic acid;
- 1-cyclopropyl-6-fluoro-7-{4-[2-fluor-o-4-((*R*)-5-hydroxymethyl-2-oxo-oxazolidin-3-yl)-phenoxymethyl]-piperidin-1-yl}-4-oxo-1,4-dihydro-quinoline-3-carboxylic acid;
- 1-cyclopropyl-6-fluoro-7-{4-[2-fluoro-4-((R)-5-hydroxymethyl-2-oxo-oxazolidin-3-yl)-phenoxymethyl]-4-hydroxy-piperidin-]-yl}-4-oxo-1,4-dihydro-[1,8]naphthyridine-3-carboxylic acid;
- 7-(4-{4-[(*R*)-5-((*S*)-2-amino-propionyloxymethyl)-2-oxo-oxazolidin-3-yl]-2-fluoro-phenoxymethyl}-4-hydroxy-piperidin-1-yl)-1-cyclopropyl-6-fluoro-4-oxo-1,4-dihydroquinoline-3-carboxylic acid;
- 1-cyclopropyl-6-fluoro-7-{4-[2-fluoro-4-((*R*)-2-oxo-5-phosphonooxymethyl-oxazolidin-3-yl)-phenoxymethyl]-4-hydroxy-piperidin-1-yl}-4-oxo-1,4-dihydroquinoline-3-carboxylic acid;
- 1-cyclopropyl-6-fluoro-7-{(*R*)-3-[2-fluoro-4-((*R*)-5-hydroxymethyl-2-oxo-oxazolidin-3-yl)-phenoxymethyl]-3-hydroxy-pyrrolidin-1-yl}-4-oxo-1,4-dihydro-quinoline-3-carboxylic acid;
- 1-cyclopropyl-6-fluoro-7-{(*S*)-3-[2-fluoro-4-((*R*)-5-hydroxymethyl-2-oxo-oxazolidin-3-yl)-phenoxymethyl]-3-hydroxy-pyrrolidin-1-yl}-4-oxo-1,4-dihydro-quinoline-3-carboxylic acid;
- 1-cyclopropyl-6-fluoro-7-{(R)-3-[2-fluoro-4-((*R*)-5-hydroxymethyl-2-oxo-oxazolidin-3-yl)-phenoxymethyl]-3-hydroxy-pyrrolidin-1-yl}-8-methoxy-4-oxo-1,4-dihydroquinoline-3-carboxylic acid;
- 1-cyclopropyl-6-fluoro-7-{(*S*)-3-[2-fluoro-4-((*R*)-5-hydroxymethyl-2-oxo-oxazolidin-3-yl)-phenoxymethyl]-3-hydroxy-pyrrolidin-1-yl}-8-methoxy-4-oxo-1,4-dihydroquinoline-3-carboxylic acid;
- 1-cyclopropyl-6-fluoro-7-{(*R*)-3-[2-fluoro-4-((*R*)-5-hydroxymethyl-2-oxo-oxazolidin-3-yl)-phenoxymethyl]-pyrrolidin-1-yl}-4-oxo-1,4-dihydro-quinoline-3-carboxylic acid;
- 1-cyclopropyl-6-fluoro-7-{(*S*)-3-[2-fluoro-4-((*R*)-5-hydroxymethyl-2-oxo-oxazolidin-3-yl)-phenoxymethyl]-pyrrolidin-1-yl}-4-oxo-1,4-dihydro-quinoline-3-carboxylic acid;
- 1-cyclopropyl-6-fluoro-7-{(*R*)-3-[2-fluoro-4-((*R*)-5-hydroxymethyl-2-oxo-oxazolidin-3-yl)-phenoxymethyl]-pyrrolidin-1-yl}-8-methoxy-4-oxo-1,4-dihydro-quinoline-3-carboxylic acid;
- 1-cyclopropyl-6-fluoro-7-{(*S*)-3-[2-fluoro-4-((*R*)-5-hydroxymethyl-2-oxo-oxazolidin-3-yl)-phenoxymethyl]-pyrrolidin-1-yl}-8-methoxy-4-oxo-1,4-dihydro-quinoline-3-carboxylic acid;
- 1-cyclopropyl-6-fluoro-7-{4-[2-fluoro-4-((*R*)-5-hydroxymethyl-2-oxo-oxazolidin-3-yl)-phenoxymethyl]-4-phosphonooxy-piperidin-1-yl}-4-oxo-1,4-dihydro-quinoline-3-carboxylic acid;
- 1-ethyl-6-fluoro-7-{4-[2-fluoro-4-((*R*)-5-hydroxymethyl-2-oxo-oxazolidin-3-yl)-phenoxymethyl]-piperidin-1-yl}-4-oxo-1,4-dihydro-quinoline-3-carboxylic acid;
- 7-(4-{4-[(*R*)-5-((*S*)-2-amino-propionyloxymethyl)-2-oxo-oxazolidin-3-yl]-2-fluoro-phenoxymethyl}-piperidin-1-yl)-1-cyclopropyl-6-fluoro-4-oxo-1,4-dihydro-quinoline-3-carboxylic acid;
- 6-fluoro-7-{4-[2-fluoro-4-((*R*)-5-hydroxymethyl-2-oxo-oxazolidin-3-yl)-phenoxymethyl]-4-hydroxy-piperidin-1-yl}-4-oxo-1,4-dihydro-quinoline-3-carboxylic acid;
- 6-fluoro-7-{4-[2-fluoro-4-((*R*)-5-hydroxymethyl-2-oxo-oxazolidin-3-yl)-phenoxymethyl]-4-hydroxy-piperidin-1-yl}-4-oxo-1,4-dihydro-[1,8]naphthyridine-3-carboxylic acid;
as well as salts thereof (and in particular pharmaceutically acceptable salts thereof).

Chimeric derivatives of formula I are suitable for the use as medicaments, particularly as antimicrobial agents, in human medicine but also in veterinary medicine in the treatment of species like pigs, ruminants, horses, dogs, cats and poultry.

Chimeric derivatives of formula I according to the present invention are also useful for the manufacture of a medicament for the treatment of infections (notably bacterial infections or protozoal infections) and disorders related to infections (notably disorders related to bacterial infections or to protozoal infections).

The compounds according to this invention are particularly active against bacteria and bacteria-like organisms. They are therefore particularly suitable in human, as well as in animals, for the prophylaxis and chemotherapy of local and systemic infections caused by these pathogens as well as disorders related to bacterial infections comprising pneumonia, otitis media, sinusitis, bronchitis, tonsillitis, and mastoiditis related to infection by *Streptococcus pneumoniae, Haemophilus influenzae, Moraxella catarrhalis, Staphylococcus aureus, Enterococcus faecalis, E. faecium, E. casseliflavus, S. epidermidis, S. haemolyticus,* or *Peptostreptococcus spp.;* pharyngitis, rheumatic fever, and glomerulonephritis related to infection by *Streptococcus pyogenes,* Groups C and G streptococci, *Corynebacterium diphtheriae,* or *Actinobacillus haemolyticum;* respiratory tract infections related to infection by *Mycoplasma pneumoniae, Legionella pneumophila, Streptococcus pneumoniae, Haemophilus influenzae,* or *Chlamydia pneumoniae;* blood and tissue infections, including endocarditis and osteomyelitis, caused by *S*. *aureus, S. haemolyticus, E. faecalis, E. faecium, E. durans,* including strains resistant to known antibacterials such as, but not limited to, beta-lactams, vancomycin, aminoglycosides, quinolones, chloramphenicol, tetracyclines and macrolides; uncomplicated skin and soft tissue infections and abscesses, and puerperal fever related to infection by *Staphylococcus aureus,* coagulase-negative staphylococci (*i.e., S. epidermidis, S. haemolyticus,* etc.), *Streptococcus pyogenes, Streptococcus agalactiae,* Streptococcal groups C-F (minute colony streptococci), viridans streptococci, *Corynebacterium minutissimum, Clostridium spp.,* or *Bartonella henselae;* uncomplicated acute urinary tract infections related to infection by *Staphylococcus aureus,* coagulase-negative staphylococcal species, or *Enterococcus spp.;* urethritis and cervicitis; sexually transmitted diseases related to infection by *Chlamydia trachomatis, Haemophilus ducreyi, Treponema pallidum, Ureaplasma urealyticum,* or *Neiserria gonorrhoeae;* toxin diseases related to infection by *S*. *aureus* (food poisoning and toxic shock syndrome), or Groups A, B, and C streptococci; ulcers related to infection by *Helicobacter pylori;* systemic febrile syndromes related to infection by *Borrelia recurrentis;* Lyme disease related to infection by *Borrelia burgdorferi;* conjunctivitis, keratitis, and dacrocystitis related to infection by *Chlamydia trachomatis, Neisseria gonorrhoeae, S. aureus, S. pneumoniae, S. pyogenes, H. influenzae,* or *Listeria* spp.; disseminated *Mycobacterium avium* complex (MAC) disease related to infection by *Mycobacterium avium,* or *Mycobacterium intracellulare;* infections caused by *Mycobacterium tuberculosis, M. leprae, M. paratuberculosis, M. kansasii,* or *M. chelonei;* gastroenteritis related to infection by *Campylobacter jejuni;* intestinal protozoa related to infection by *Cryptosporidium* spp.; odontogenic infection related to infection by viridans streptococci; persistent cough related to infection by *Bordetella pertussis;* gas gangrene related to infection by *Clostridium perfringens* or *Bacteroides* spp.; and atherosclerosis or cardiovascular disease related to infection by *Helicobacter pylori* or *Chlamydia pneumonia.*

Compounds of formula I according to the present invention are further useful for the preparation of a medicament for the treatment of infections that are mediated by bacteria such as *E. coli, Klebsiella pneumoniae* and other Enterobacteriaceae, *Acinetobacter spp., Stenothrophomonas maltophilia, Neisseria meningitidis, Bacillus cereus, Bacillus anthracis, Corynebacterium spp., Propionibacterium acnes* and bacteroide *spp.* In addition, compounds of formula I according to the present invention are further useful for the preparation of a medicament for the treatment of infections that are mediated by *Clostridium difficile.*

Compounds of formula I according to the present invention are further useful to treat protozoal infections caused by *Plasmodium malaria, Plasmodium falciparum, Toxoplasma gondii, Pneumocystis carinii, Tripanosoma brucei* and *Leishmania spp.*

The preceding lists of pathogens arc to be interpreted merely as examples and in no way as limiting.

As well as in humans, bacterial infections can also be treated in other species like pigs, ruminants, horses, dogs, cats and poultry.

Therefore, the compounds of formula I or their pharmaceutically acceptable salts can be used for the preparation of a medicament, and are suitable, for the prevention or treatment of bacterial infections (notably those caused by the pathogens mentioned in the lists above).

The compounds of formula I and their pharmaceutically acceptable salts can be used as medicaments, e.g. in the form of pharmaceutical compositions for enteral or parental administration.

The production of the pharmaceutical compositions can be effected in a manner which will be familiar to any person skilled in the art (see for example Mark Gibson, Editor, Pharmaceutical Preformulation and Formulation, IHS Health Group, Englewood, CO, USA, 2001; Remington, The Science and Practice of Pharmacy, 20th Edition, Philadelphia College of Pharmacy and Science) by bringing the described compounds of formula I or their pharmaceutically acceptable salts, optionally in combination with other therapeutically valuable substances, into a galenical administration form together with suitable, non-toxic, inert, therapeutically compatible solid or liquid carrier materials and, if desired, usual pharmaceutical adjuvants.

Another aspect of the invention concerns a use of a compound of formula I or of a pharmaceutically acceptable salt thereof for the manufacture of a medicament intended for the prevention or treatment of an infection comprising the administration to the patient of a pharmaceutically active amount of a compound according to formula I or of a pharmaceutically acceptable salt thereof.

Moreover, the compounds of formula I may also be used for cleaning purposes, e.g. to remove pathogenic microbes and bacteria from surgical instruments or to make a room or an area aseptic. For such purposes, the compounds of formula I could be contained in a solution or in a spray formulation.

Any reference to a compound of formula I, I₅₁, I₅₂, I₆, I_{D} or I_{PDG} is to be understood as referring also to a salt (especially a pharmaceutically acceptable salt) of a compound of formula I, I₅₁, I₅₂, I₆, I_{D} or I_{PDG} respectively, as appropriate and expedient. Besides, any preferences indicated for the compounds of formula I (whether for the compounds themselves, salts thereof, compositions containing the compounds or salts thereof, uses of the compounds or salts thereof, etc.) apply *mutatis mutandis* to compounds of formula I_{CE}, the compounds of formula I₅, the compounds of formula I₅₁, the compounds of formula I₅₂, the compounds of formula I₆, the compounds of formula I_{D} and the compounds of formula I_{PDG}.

According to the invention, the compounds of formula I can be prepared by the process described hereafter.

### Preparation of the compounds of formula I

### Abbreviations:

The following abbreviations are used throughout the specification and the examples:
- AcOH: acetic acid
- AD-mix α: 1,4-*bis*(dihydroquinine)phthalazine, K₃Fe(CN)₆, K₂CO₃ and K₂OsO₄.2H₂O
- AD-mix β: 1,4-*bis*(dihydroquinidine)phthalazine, K₃Fe(CN)₆, K₂CO₃ and K₂OsO₄.2H₂O
- Alloc: allyloxycarbonyl
- aq.: aqueous
- BnBr: benzyl bromide
- Boc: *tert*-butoxycarbonyl
- *t*-BuOK: potassium *tert*-butylate
- Cbz: benzyloxycarbonyl
- DBU: 1,8-diazabicyclo[5.4.0]undec-7-ene
- DCM: dichloromethane
- DIAD: diisopropyl azodicarboxylate
- DIPEA: *N,N*-diisopropylethylamine
- DMA: dimethylacetamide
- DMAP: 4-dimethylaminopyridine
- DMF: *N,N*-dimethylformamide
- DMSO: dimethylsulfoxide
- EA: ethyl acetate
- EDC: 1-(dimethylaminopropyl)-3-ethylcarbodiimide hydrochloride
- ESI: Electron Spray Ionisation
- ether or Et₂O: diethyl ether
- FC: flash chromatography
- h: hour
- Hex: *n*-hexane
- MeCN: acetonitrile
- MCPBA: *meta*-chloroperbenzoic acid
- MeOH: methanol
- MS: Mass Spectroscopy
- NaOMe: sodium methylate
- NMP: *N*-methylpyrrolidinone
- org.: organic
- Pd/C or Pd(OH)₂/C: palladium or dihydroxypalladium on charcoal
- PPh₃: triphenylphosphine
- rt: room temperature
- sat.: saturated
- SiO₂: silica gel
- TBDMSCI: *tert*-butyldimethylsilyl chloride
- TEA: triethylamine
- TFA: trifluoroacetic acid
- THF: tetrahydrofuran
- TMSCI: trimethylsilyl chloride

### General preparation routes:

The novel compounds of formula I can be manufactured in accordance with the present invention by
a) reacting the compound of formula II 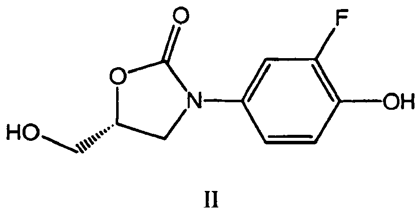 with a compound of formula III 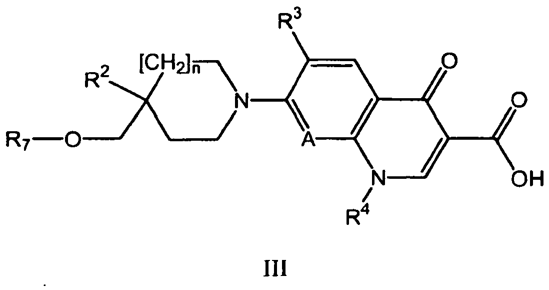 wherein n, A, R³ and R⁴ are as defined in formula I and R⁷ is (C₁-C₃)alkylsulfonyl (e.g. methylsulfonyl), trifluoromethylsulfonyl or arylsulfonyl (e.g. phenyl- or *p*-tolyl-sulfonyl) and R² is OH or H, or R² and R⁷ together form a bond (i.e. R² and OR⁷ form, together with the carbon atoms that carry them, an epoxide ring), preferably between about 10°C and 100°C (more preferably between about 40°C and 80°C), in the presence of an inorganic base such as K₂CO₃ or an organic base such as TEA in an organic solvent (e.g. DMF);
   or
b) reacting a compound of formula IV 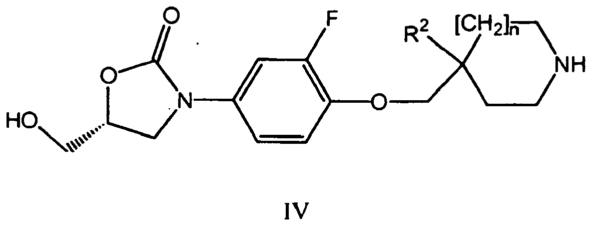 wherein n is as defined in formula I and R² is H or OH,
   with a compound of formula V 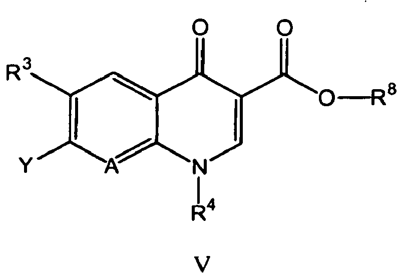 wherein A, R³ and R⁴ are as defined in formula I, Y is halogen and R⁸ is hydrogen, BF₂ or B(OC(=O)(C₁-C₄)alkyl)₂, (C₁-C₅)alkyl (*e*.*g*. methyl, ethyl, *n*-propyl, *iso-*propyl or *tert*-butyl), allyl, aryl-(C₁-C₅)alkyl (*e.g*. benzyl, *p*-nitrobenzyl or *p-*methoxybenzyl), tri-(C₁-C₅)alkylsilyl (*e.g*. trimethylsilyl or *tert*-butyldimethylsilyl) or diaryl-(C₁-C₅)alkylsilyl (*e.g*. *tert*-butyldiphenylsilyl), preferably between about 10°C and 100°C, more preferably between about 40°C and 80°C in the presence of an organic base, such as TEA or DIPEA, in an organic solvent, e.g. NMP;
   or
c) converting a compound of formula I wherein R¹ is OH into a compound of formula I wherein R¹ is OPO₃H₂ or OCOR⁵, R⁵ being the residue of a naturally occurring amino acid, of the enantiomer of a naturally occurring amino acid or of dimethylaminoglycine;
   or
d) converting a compound of formula I_{PG} 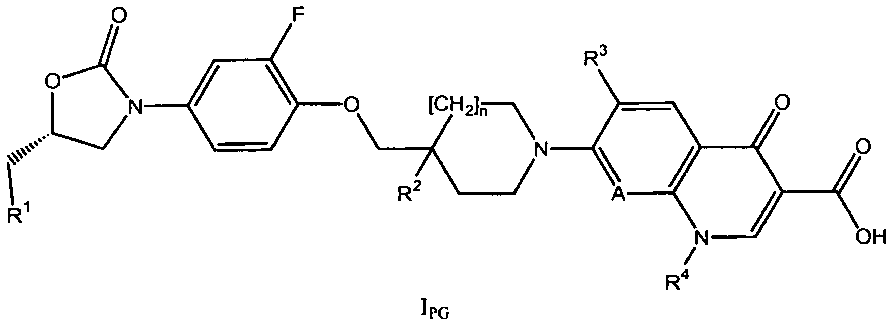 wherein R¹ is OPG¹ (PG¹ being a protecting group for an alcohol function), R² is OH, and n, R³, R⁴ and A have the same meaning as in formula I, into a compound of formula I wherein R² is OPO₃H₂ and subsequently removing the protecting group PG¹, examples of suitable protecting groups PG¹ being alkylsilyl or diarylalkylsilyl groups such as trimethylsilyl, tert-butyldimethylsilyl or tert-butyldiphenylsilyl (strategies to introduce these protecting groups and to remove them have been summarized in Protecting groups, Kocienski, P.J., Thieme (1994));
   or
e) converting a compound of formula VI 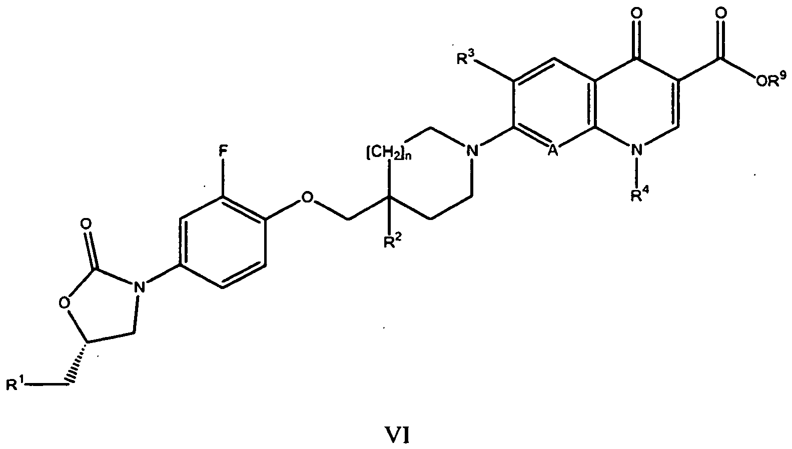 wherein R⁹ is (C₁-C₅)alkyl (*e.g.* methyl, ethyl, *n*-propyl, *iso*-propyl or *tert*-butyl), aryl-(C₁-C₅)alkyl (e.g. benzyl, *p*-nitrobenzyl or p-methoxybenzyl), allyl, tri-(C₁-C₅)alkylsilyl (e.g. trimethylsilyl or *tert*-butyldimethylsilyl) or diaryl-(C₁-C₅)alkylsilyl (e.g. *tert*-butyldiphenytsilyl) and n, A, R¹, R², R³ and R⁴ are as defined in formula I into the corresponding compound of formula I by hydrolysis, saponification or hydrogenolysis (e.g. as reviewed in Protecting groups, Kocienski, P.J., Thieme (1994)).

Concerning the above process, the following should be noted:
❖ regarding variant a), the compound of formula III could also be replaced by an ester thereof, i.e. a compound of formula III_{E} 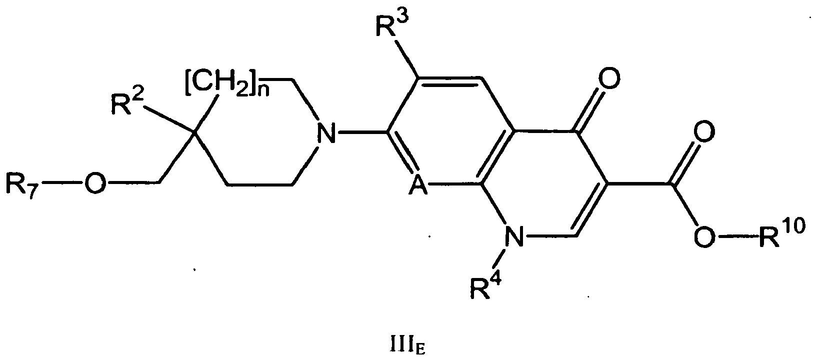 wherein n, A, R², R³, R⁴ and R⁷ are as defined in formula III and R¹⁰ represents alkyl, allyl or arylalkyl, in which case an ester deprotection step would follow the reaction of the compound of formula III_{E} with the compound of formula II (general methods to perform the ester deprotection step have been reviewed in Protecting groups, Kocienski, P.J., Thieme (1994));
❖ regarding variant a), the compound of formula II could also be replaced by a silyl ether thereof, i.e. a compound of formula II_{PG} 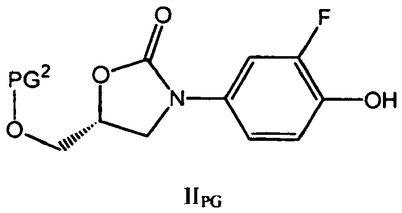 wherein PG² represents a silyl protecting group for an alcohol function such as a tri-(C₁-C₅)alkylsilyl (e.g. trimethylsilyl or *tert*-butyldimethylsilyl) or diaryl-(C₁-C₅)alkylsilyl (e.g. *tert*-butyldiphenylsilyl), in which case a deprotection step would follow the reaction of the compound of formula III or III_{E} with the compound of formula II_{PG} (general methods to perform such reactions have been reviewed in Protecting groups, Kocienski, P.J., Thieme (1994)), it being understood that when R² is H, the coupling between compound of formula II_{PG} and the compound of formula III or III_{E} can also be performed under Mitsunobu conditions as described in Synthesis (1981), 1, 1-28, and notably conditions wherein the reaction is carried out in the presence of DIAD and PPh₃;
❖ regarding variant b), the compound of formula IV could also be replaced by a compound of formula IVₚ 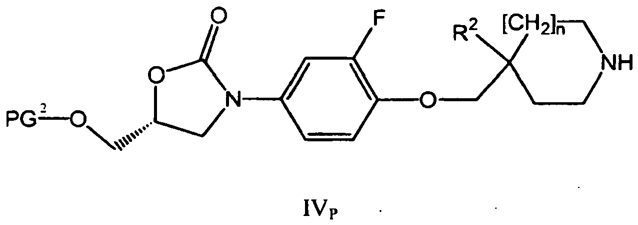 wherein n and R² are as defined in formula IV and PG² represents a protecting group for an alcohol function (e.g. an alkylsilyl or diarylalkylsilyl group such as trimethylsilyl, *tert*-butyldimethylsilyl or *tert*-butyldiphenylsilyl), in which case the appropriate deprotection step would follow the reaction of the compound of formula IV_{P} with the compound of formula V (general methods to perform such reactions have been reviewed in Protecting groups, Kocienski, P.J., Thieme (1994));
❖ regarding variant b), when R⁸ is not H, an additional ester deprotection step is required (general methods to perform such reactions have been reviewed in Protecting groups, Kocienski, P.J., Thieme (1994)), except for the cases wherein R⁸ is BF₂ or B(OC(=O)(C₁-C₄)alkyl)₂ where the hydrolysis takes place already during the acidic work-up;
❖ regarding variant c), the compound of formula I wherein R¹ is OH can be replaced by a compound of formula VI wherein R¹ is OH and R² is H or OH, in which case an additional ester deprotection step is required (general methods to perform such reactions have been reviewed in Protecting groups, Kocienski, P.J., Thieme (1994));
❖ concerning variants c) and d):
   ○ compounds of formula I wherein R¹ or R² is OPO₃H₂ can be obtained by deprotection of the corresponding compounds wherein R¹ or R² is OPO(OR)₂ and R is allyl or benzyl (according to the nature of R, various methods for deprotection may be used as reviewed in Protecting Groups, Kocienski, P., J., Thieme (1994), like for example catalytic hydrogenation over a noble catalyst such as palladium or hydrolysis with hydrobromic acid in a solvent such as AcOH when R is benzyl);
   ○ compounds of formula I wherein R¹ is OCOR⁵ can be obtained for example by reaction of compounds of formula I wherein R¹ is OH or compounds of formula VI wherein R¹ is OH and R² is H or OH with dimethylaminoglycine or a *N*-protected amino acid followed by deprotection of the amino group under standard conditions known to one skilled in the art (an additional ester deprotection step being required in the case of a reaction with a compound of formula VI).

The compounds of formula I obtained according to the abovementioned general preparation methods may then, if desired, be converted into their salts, and notably into their pharmaceutically acceptable salts.

Besides, whenever the compounds of formula I are obtained in the form of mixtures of enantiomers, the enantiomers can be separated using methods known to one skilled in the art (e.g. by formation and separation of diastereomeric salts or by chromatography over a chiral stationary phase). Whenever the compounds of formula I are obtained in the form of mixtures of diasteromers they may be separated by an appropriate combination of silica gel chromatography, HPLC and crystallization techniques.

### Preparation of the various synthesis intermediates:

### Preparation of the compound of formula II

The compound of formula II can be obtained by hydrogenation of compound VII 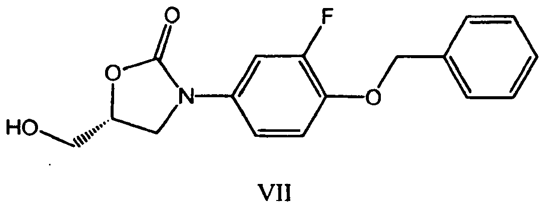 over a noble catalyst such as palladium or platinum on charcoal in a solvent such as THF, MeOH or AcOEt between 0°C and 40°C or by hydrolysis of in presence of a solution of HBr in water or AcOH between 0°C and 80°C in a solvent such as AcOH.

### Preparation of the compounds of formula III

The compounds of formula III can be prepared as summarized in Scheme 1 hereafter.

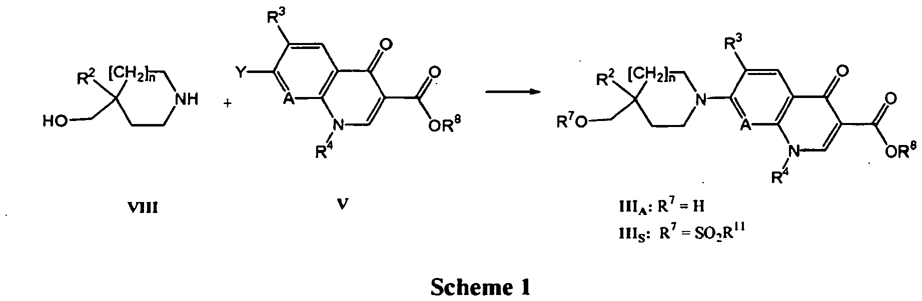

In Scheme 1, R⁸ is H, alkyl, allyl or arylalkyl, and the other symbols are as before.

Compounds of formula III_{S} wherein R² is H or OH, R⁷ is SO₂R¹¹, R¹¹ being alkyl, trifluoromethyl or aryl like phenyl or *p*-tolyl are obtained (Scheme 1) from compounds of formula III_{A} wherein R⁷ is H by reaction with the corresponding sulfonyl chlorides in presence of an organic base such as TEA in a solvent such as DCM or THF between -10°C and 50°C. Compounds of formula III_{A} are prepared by reaction of the compounds of formula V with the piperidines of formula VIII in the presence of an organic base such as TEA or DIPEA between 40°C and 100°C in a solvent such as THF, DMF or NMP. If R⁸ is benzyl, the carboxylic acid of formula III_{S} is liberated according to standard procedures as described in Protecting groups, Kocienski, P.J., Thieme (1994) (e.g. hydrogenation over Pd/C).

The compounds of formula III wherein R² and R⁷ together form a bond, i.e. the compounds of formula III_{O} 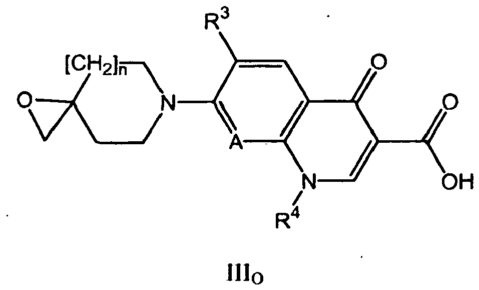 can be prepared by intramolecular cyclisation of the compounds of formula III_{S} wherein R² is OH in the presence of an organic base (e.g. TEA) or an inorganic base (e.g. K₂CO₃ or an alkali methylate such as NaOMe or an alkali hydride such as NaH).

### Preparation of the compounds of formula IV

The compounds of formula IV can be prepared as summarized in Scheme 2 hereafter.

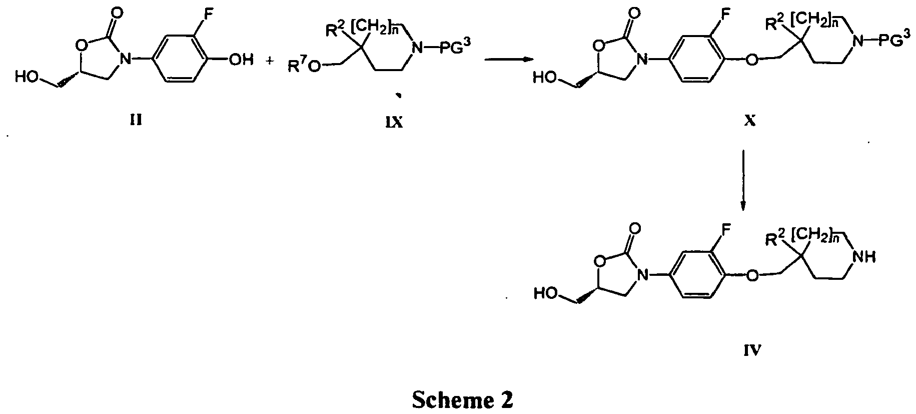

The compounds of formula IV can be obtained by deprotecting a compound of formula X wherein PG³ represents a nitrogen protecting group such as alkoxycarbonyl (e.g. Boc), benzyloxycarbonyl (e.g. Cbz), Alloc or benzyl. General methods to perform such protection/deprotection sequences of secondary nitrogen atoms have been reviewed in Protecting groups, Kocienski, P.J., Thieme (1994).

The compounds of formula X wherein R² is OH are obtained by reacting a compound of formula II with a compound of formula IX wherein either R² is OH and R⁷ is SO₂R¹¹, R¹¹ being alkyl, trifluoromethyl or aryl like phenyl or *p*-tolyl, or R² and R⁷ together form a bond (epoxide). Said epoxide can be obtained from compounds of formula IX, wherein R² is OH and R⁷ is SO₂R¹¹ upon treatment with either an organic base such as TEA, pyridine or DBU or an inorganic base such as K₂CO₃ in a solvent such as THF, ether or DCM between -10°C and 40°C. Compounds of formula X, wherein R² is H are obtained by reacting a compound of formula II with a compound of formula IX wherein R⁷ is SO₂R¹¹. Compounds of formula IX are either commercially available (e.g. compounds of formula IX wherein PG³ = Boc, n = 0 and R² and R⁷ form an epoxide or wherein PG³ = Boc, n = 1 and R² = R⁷ = OH) or made as explained later on. Alternatively, compounds of formula X can be obtained by reaction of the compound of formula II with compounds of formula IX wherein R⁷ is H under Mitsunobu conditions.

### Preparation of the compounds of formula V

Compounds of formula V wherein R⁸ is H and Y halogen are commercially available (e.g. compounds wherein R³ = F, R⁴ = cyclopropyl and A = CH, CF or COMe, or R³ = F, R⁴ = Et and A = CH or CF, or R³ = F, R⁴ = cyclopropyl and A = N). Compounds of formula V wherein R⁸ is BF₂ or B(OC(=O)(C₁-C₄)alkyl)₂ are obtained from compounds of formula V wherein R⁸ is H according to WO 88/07998.

### Preparation of the compounds of formula VI

The compounds of formula VI can be obtained by coupling compounds of formula IV or, alternatively, compounds of formula IV_{P} as previously defined with compounds of formula V as previously defined except that R⁸ represents (C₁-C₅)alkyl (e.g. methyl, ethyl, *n*-propyl, *iso*-propyl or *tert*-butyl), aryl-(C₁-C₅)alkyl (*e.g*. benzyl, *p*-nitrobenzyl or p-methoxybenzyl), allyl, tri-(C₁-C₅)alkylsilyl (e.g. trimethylsilyl or *tert-*butyldimethylsilyl) or diaryl-(C₁-C₅)alkylsilyl (e.g. *tert*-butyldiphenylsilyl), under the same conditions as those described for the reaction of the compounds of formula IV with the compounds of formula V. If the compounds of formula IV_{P} are used, the deprotection step can be carried out after the coupling reaction.

### Preparation of the compound of formula VII

The compound of formula VII can be obtained according to WO 2004/096221.

### Preparation of the compounds of formula VIII

Compounds of formula VIII are either commercially available (R² = H) or obtained by deprotection of compounds of formula IX (R² = OH and R⁷ = H), for example by treatment of the corresponding Boc protected compounds with TFA or by hydrogenation of the corresponding Cbz protected compounds over Pd/C.

### Preparation of the compounds of formula IX

The compounds of formula IX can be prepared from the methylidene derivatives of formula XI as summarized in Scheme 3 hereafter.

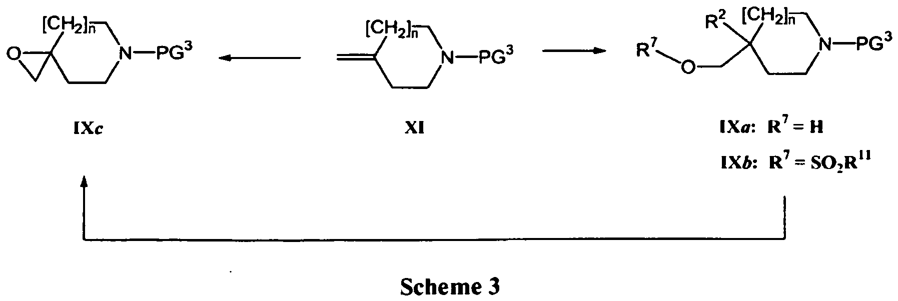

The compounds of formula IX*b*, i.e. the compounds of formula IX wherein R² is H or OH and R⁷ is SO₂R¹¹, are prepared from the corresponding compounds of formula IXa wherein R⁷ is H using the same methods used for the conversion of compounds of formula III_{A} into compounds of formula III_{S}. Compounds of formula IX*a* either are commercially available (R² = H) or are obtained from the known methylidene derivatives of formula XI (e.g. those wherein n = 0 and PG³ = benzyl, Boc or benzyloxycarbonyl - see EP 241206 and EP 550025; or those wherein n = 1 and PG³ = benzyl, Boc which are commercial) either by osmium tetroxide catalyzed cis-dihydroxylation or by its asymmetric version (Sharpless dihydroxylation using AD-mix α or β) as described in J. Am. Chem. Soc. (1988), 110, 1968 (R² = OH).

Compounds of formula IX*c*, i.e. the compounds of formula IX wherein R² and R⁷ together form a bond (epoxide), are obtained either by intramolecular ring closure of compounds of formula IX*b* with an inorganic base such as K₂CO₃ or NaH or an organic base such as TEA or DBU, or by epoxidation of the methylidene double bond with a peracid such as MCPBA. Alternatively, compounds of formula IX*c* can also be obtained by reaction of the corresponding oxo derivatives (commercial when n = 0 or 1 and PG³ = Cbz or Boc) with trimethylsulfoxonium iodide or trimethylsulfonium iodide in presence of an alkali hydroxide such as KOH in a polar solvent such as MeCN between 20 and 100°C (as described in J. Am. Chem. Soc. (1965), 87, 1353-1364 and Tetrahedron Lett. (1987), 28, 1877-1878).

The following examples further illustrate the preparation of the pharmacologically active compounds of the invention but do not limit the scope thereof.

### EXAMPLES

All temperatures are stated in °C. All analytical and preparative HPLC investigations on non-chiral phases are performed using RP-C18 based columns. Analytical HPLC investigations are performed on two different instruments with cycle-times of ~2.5 min and -3.5 min respectively. Unless otherwise stated, the values indicated for MS correspond to the main peaks ((M+H)⁺ with a variation of +/- 0.5 unit). In NMR spectra, coupling constants J are given in Hz.

### Standard work-up procedure:

After dilution in the appropriate org. solvent (see corresponding Example text), the org. phase is separated and sequentially washed with water and brine. In case of reaction performed in a water soluble solvent (e.g. MeOH, THF or DMF), the combined aq. layers are back-washed with the same solvent used to perform the workup. The combined org. phases are dried over MgSO₄ and filtered. The filtrate is evaporated under reduced pressure.

### Standard chromatography procedure:

The crude material is dissolved in the minimum of eluent (see corresponding Example text) and chromatographed over SiO₂. The relevant fractions were pooled and evaporated under reduced pressure.

### Example 1: 1-cyclopropyl-6-fluoro-7-{4-[2-fluoro-4-((R)-5-hydroxymethyl-2-oxo-oxazolidin-3-yl)-phenoxymethyl]-4-hydroxy-piperidin-1-yl}-4-oxo-1,4-dihydro-quinoline-3-carboxylic acid:

### 1.i. (R)-3-(3-fluoro-4-hydroxy-phenyl)-5-hydroxymethyl-oxazolidin-2-one:

A solution of (*R*)-3-(4-benzyloxy-3-fluoro-phenyl)-5-hydroxymethyl-oxazolidin-2-one (6.34 g, prepared according to WO 2004/096221) in THF/MeOH (1:1; 200 ml) was hydrogenated over Pd/C 10% (1 g) overnight. The catalyst was filtered off, the filtrate evaporated under reduced pressure and the residue stirred in EA. The crystals were collected by filtration, affording 3.16 g (70% yield) of a colourless solid.

¹H NMR (DMSO_{d6}; δ ppm): 3.5 (m, 1H), 3.64 (m, 1H), 3.74 (dd, J = 8.8, 6.4, 1 H), 3.99 (t, J = 8.8, 1 H), 4.64 (m, 1H), 5.16 (t, J = 5.6, 1H), 6.93 (dd, J = 9.7, 8.8, 1 H), 7.08 (ddd, J = 8.8, 2.6, 1.2, 1 H), 7.45 (dd, J = 13.5, 2.6, 1 H), 9.66 (s, 1H).

MS (ESI): 228.1.

### 1.ii. 4-[2-fluoro-4-((R)-5-hydroxymethyl-2-oxo-oxazolidin-3-yl)-phenoxymethyl]-4-hydroxy-piperidine-1-carboxylic acid benzyl ester:

A solution of intermediate 1.i (1.27g) and 1-oxa-6-aza-spiro[2.5]octane-6-carboxylic acid benzyl ester (1.60 g; prepared according to US 4244961) were dissolved in DMF (15 ml) and treated with Na₂CO₃ (1.16 g). The mixture was heated at 100°C overnight.

The residue obtained after workup (DCM) was stirred in EA, and the solid was collected by filtration and sequentially washed with EA and Hex, affording 2.52 g (94.5% yield) of a beige solid.

¹H NMR (DMSO_{d6}; δ ppm): 1.57 (m, 4 H), 3.14 (m, 2 H), 3.54 (m, 1H), 3.64 (m, 1H), 3.79 (m, 5 H), 4.03 (t, J = 9.1, 1H), 4.66 (m, 1H), 4.78 (s, 1H), 5.05 (s, 2 H), 5.16 (t, J=5.6, 1H), 7.18 (m, 2H), 7.32 (m, 5H), 7.55 (d, J = 12, 1H).

MS (ESI): 475.0.

### 1.iii. (R)-3-[3-fluoro-4-(4-hydroxy-piperidin-4-ylmethoxy)-phenyl]-5-hydroxymethyl-oxazolidin-2-one:

A suspension of intermediate 1.ii (2.5 g) in EA/MeOH (1:1; 100 ml) was hydrogenated over Pd/C for 48 h. The suspension was heated at 40°C and the catalyst was filtered off.

The filtrate was evaporated under reduced pressure affording 1.61 g (89% yield) of a yellow powder.

¹H NMR (DMSO_{d6}; δ ppm): 1.4-1.63 (m, 4 H), 2.67 (m, 2 H), 2.83 (m, 2 H), 3.53 (dd, J = 4.0 and 12.0, 1H); 3.66 (dd, J = 3.3 and 12.0, 1H), 3.71 (s, 2H); 3.80 (m, 1H), 4.05 (t, J = 9.0, 1 H), 4.48 (s, 1H), 4.68 (m, 1H), 5.20 (s, 1H), 7.20 (m, 2 H), 7.57 (d, 1H).

MS (ESI): 341.5.

### 1.iv. 1-cyclopropyl-6-fluoro-7-{4-[2-fluoro-4-((R)-5-hydroxymethyl-2-oxo-oxazolidin-3-yl)-phenoxymethyl]-4-hydroxy-piperidin-1-yl}-4-oxo-1,4-dihydro-quinoline-3-carboxylic acid:

A solution of intermediate 1.iii (200 mg), 7-chloro-1-cyclopropyl-6-fluoro-1,4-dihydro-4-oxo-3-quinolinecarboxylic acid boron diacetate complex (241 mg; prepared according to WO 88/07998) and DIPEA (100 µl) in NMP (2 ml) was stirred at 85°C for 5 h. The reaction mixture was evaporated under reduced pressure and the residue was taken up in *5M* HCl in MeOH (3 ml) and stirred. The resulting solid was collected by filtration and washed with MeOH to afford 230 mg (67% yield) of a yellow solid.

¹H NMR (DMSO_{d6}; δ ppm): 1.66-1.35 (m, 4 H), 1.75 (d, J = 12.8, 2H), 1.95 (m, 2 H), 3.33 (t broad, J = 11.0, 2 H), 3.57 (m, 3 H), 3.67 (dd, J = 12.3, 3.3, 1H), 3.83 (m, 2 H), 3.92 (s, 2 H), 4.06 (t, J = 9.0, 1 H), 4.69 (m, 1H), 7.24 (m, 2 H), 7.60 (m, 2 H), 7.90 (d, J = 13.3, 1H), 8.66 (s, 1H).

MS (ESI): 585.9.

### Example 2: 1-cyclopropyl-6-fluoro-7-{4-[2-fluoro-4-((R)-5-hydroxymethyl-2-oxo-oxazolidin-3-yl)-phenoxymethyl]-piperidin-1-yl}-4-oxo-1,4-dihydro-quinoline-3-carboxylic acid:

### 2.i. (R)-3-(4-benzyloxy-3-fluoro-phenyl)-5-(tert-butyl-dimethyl-silanyloxymethyl)-oxazolidin-2-one:

A solution of TBDMSCl (3.77 g) in DCM (5 ml) was added dropwise at 0°C to a solution of (*R*)-3-(4-benzyloxy-3-fluoro-phenyl)-5-hydroxymethyl-oxazolidin-2-one (6.35 g; prepared according to WO 2004/096221) and imidazole (2.04 g) in DMF (15 ml). After stirring at rt for 16 h, the reaction mixture concentrated *in vacuo.* The residue was taken up in DCM and sequentially washed with 1*N* HCl, sat. NaHCO₃ aq. and brine, dried over MgSO₄, filtered and concentrated to give 8.41 g (97% yield) of a colourless solid.

¹H NMR (DMSO_{d6}; δ ppm): 0.04 (6H, s); 0.79 (9H, s); 3.69-3.78 (2H, m), 3.86 (1H, dd, J = 3 and J = 12); 4.07 (1H, t, J = 9); 4.69-4.77 (1H, m); 5.15 (2H, s); 7.15-7.21 (1H, m); 7.25 (1H, t, J = 9); 7.30-7.36 (1H, m); 7.37-7.50 (4H, m); 7.57 (1H, dd, J = 3 and J = 14).

### 2.ii. (R)-5-(tert-butyl-dimethyl-silanyloxymethyl)-3-(3-fluoro-4-hydroxy-phenyl)-oxazolidin-2-one:

A solution of intermediate 2.i (7.22 g) in THF/MeOH (1:1; 150 ml) was hydrogenated over 10% Pd/C (150 mg) for 3 h. The catalyst was filtered off and the filtrate concentrated *in vacuo* affording 5.51 g (96% yield) of a colourless solid.

¹H NMR (DMSO_{d6}; δ ppm): 0.04 (6H, s); 0.80 (9H, s); 3.69-3.78 (2H, m), 3.86 (1H, dd, J = 3 and J = 12); 4.07 (1H, t, J = 9); 4.68-4.75 (1H, m); 6.94 (1H, t, J = 9); 7.04-7.10 (1H, m); 7.45 (1H, dd, J = 3 and J = 14); 9.65 (1H, s).

MS (ESI): 342.2.

### 2.iii. 4-{4-[(R)-5-(tert-butyl-dimethyl-silanyloxymethyl)-2-oxo-oxazolidin-3-yl]-2-fluoro-phenoxymethyl}-piperidine-1-carboxylic acid tert-butyl ester:

A suspension of 4-hydroxymethylpiperidine-1-carboxylic acid tert-butyl ester (200 mg; commercial), intermediate 2.ii (317 mg) and PPh₃ (365 mg) in THF (5 ml) was treated dropwise over 90 min with DIAD (0.294 ml). After stirring overnight at rt, the reaction mixture was worked up (toluene/Hex 1:2) and chromatographed (Hex/EA 2:1), affording 351 mg (70% yield) of an off-white solid.

MS (ESI): 539.2.

### 2.iv. (R)-3-[3-fluoro-4-(piperidin-4-ylmethxy)-phenyl]-5-hydroxymethyl-oxazolidin-2-one:

A solution of intermediate 2.iii (351 mg) in MeOH (2 ml) was treated with 5M HCl in MeOH (1 ml) and stirred at rt for 3 h. The resulting solid was collected by filtration and washed with MeOH (5 ml), affording 180 mg (85% yield) of a colourless solid.

¹H NMR (DMSO_{d6}; δ ppm): 1.48 (2H, m); 1.89 (2H, m); 2.05 (1H, m); 2.88 (2H, t, J = 10); 3.10 (2H, m); 3.55 (1H, m); 3.63 (1H, m); 3.78 (1H, dd, J = 6.4 and J = 8.8); 3.91 (2H, d, J = 6.2); 4.02 (1H, t, J = 8.8); 4.66 (1H, m); 5.19 (1H, t, J = 5.6); 7.20 (2H, m); 7.56 (1H, dd, J = 2.35 and J = 14).

MS (ESI): 325.5.

### 2.v. 1-cyclopropyl-6-fluoro-7-{4-[2-fluoro-4-((R)-5-hydroxymethyl-2-oxo-oxazolidin-3-yl)Phenoxymethyl]-piperidin-1-yl}-4-oxo-1,4-dihydro-quinoline-3 carboxylic acid:

The title compound was obtained as a colourless powder in 12 % yield, starting from intermediate 2.iv (177 mg) and 7-chloro-1-cyclopropyl-6-fluoro-1,4-dihydro-4-oxo-3-quinolinecarboxylic acid boron diacetate complex (205 mg) and following the procedure of Example 1, step 1.iv.

¹H NMR (DMSO_{d6}; δ ppm): 1.13-1.33 (m, 4 H), 1.45-1.6 (m, 2 H), 1.94 (dl, J = 12.0, 2 H), 2.04 (m, 1 H), 2.98 (t, J = 12.0, 2 H), 3.50-3.69 (m, 2 H), 3.73-3.89 (m, 4 H), 3.98 (d, J = 6.2, 2 H), 4.02 (t, J = 9.3, 1 H), 4.66 (m, 1 H), 5.18 (t, J = 5.6, 1 H), 7.21 (m, 2 H), 7.56 (m, 2 H), 7.88 (d, J = 13.5, 1H), 8.64 (s, 1 H).

MS (ESI): 570.2.

### Example 3: 1-cyclopropyl-6-fluoro-7-{4-[2-fluoro-4-((R)-5-hydroxymethyl-2-oxo-oxazolidin-3-yl)-phenoxymethyl]-4-hydroxy-piperidin-1-yl}-4-oxo-1,4-dihydro-[1,8]naphthyridine-3-carboxylic acid:

A solution of 7-chloro-1-cyclopropyl-6-fluoro-1,4-dihydro-4-oxo-1,8-naphthyridine-3-carboxylic acid (166 mg; commercial) and intermediate 1.iii (200 mg) in NMP (5 ml) was treated with TEA (0.32 ml) and TMSCl and heated at 85°C for 5 h. The reaction mixture was evaporated under reduced pressure and the residue was taken up in 5*M* HCl in MeOH (3 ml) and stirred for 30 min. The solution was evaporated under reduced pressure and the residue was taken up in EA. The resulting solid was collected by filtration and washed with EA, affording 271 mg (78% yield) of a yellow solid.

¹H NMR (DMSO_{d6}; δ ppm): 0.89-1.27 (4H, m); 1.78 (2H, d, J = 12.8); 1.90-2.04 (2H, m); 3.53-3.88 (6H, m); 3.88 (2H, s), 4.06 (1H, t, J = 9.0), 4.42 (2H, d broad, J = 13.2), 4.44 (1H, m); 7.11 (2H, m); 7.55 (1H, d, J = 14.5); 8.05 (1H, d, J = 13.5); 8.60 (1H,s). MS (ESI): 586.8.

### Example 4: 7-(4-{4-[(R)-5-((S)-2-amino-propionyloxymethyl)-2-oxo-oxazolidin-3-yl]-2-fluoro-phenoxymethyl}-4-hydroxy-piperidin-1-yl)-1-cyclopropyl-6-fluoro-4-oxo-1,4-dihydro-quinoline-3-carboxylic acid hydrochloride:

### 4.i. 1-cyclopropyl-6-fluoro-7-{4-[2-fluoro-4-((R)-5-hydroxymethyl-2-oxo-oxazolidin-3-yl)-phenoxymethyl]-4-hydroxy-piperidin-l-yl}-4-oxo-1,4-dihydro-quinoline-3-carboxylic acid benzyl ester:

A suspension of intermediate 1.iv (300 mg) and K₂CO₃ (77.8 mg) in DMF (10 ml) was treated with BnBr (82 µl) and stirred at 80°C for 2 days. The solvents were removed under reduced pressure. The residue was worked up (DCM) and purified by chromatography (DCM/MeOH 95:5) affording 219 mg (63% yield) of a white powder.

¹H NMR (DMSO_{d6}; δ ppm): 1.0-1.25 (4H, m); 1.71 (2H, dd, J = 0.6 and J = 12.9); 1.92 (2H, m); 3.25 (2H, m); 3.50 (3H, m); 3.65 (2H, m); 3.78 (1H,dd, J = 6.4 and J = 8.8); 3.89 (2H, s); 4.03 (1H, t, J = 9.1); 4.65 (1H, m); 4.82 (1H, s); 5.17 (1H, t, J = 5.6); 5.25 (2H, s); 7.21 (2H, m); 7.35-7.60 (7H, m); 7.74 (1H, d, J = 13.5); 8.45 (1H, s).

MS (ESI): 676.2.

### 4.ii. 7-(4-{4-[(R)-5-((S)-2-tert-butoxycarbonylamino-propionyloxymethyl)-2-oxo-oxazolidin-3-yl]-2-fluoro-phenoxymethyl}-4-hydroxy-piperidin-1-yl)-1-cyclopropyl-6-fluoro-4-oxo-1,4-dihydro-quinoline-3-carboxylic acid benzyl ester:

A solution of intermediate 4.i (219 mg) in DMF (3 ml) was treated with Boc-L-Ala-OH (79.7 mg), EDC (81 mg) and DMAP (20 mg). The reaction was stirred at rt for 2 h. The DMF was evaporated under reduced pressure and the residue was purified by chromatography (DCM/MeOH 95:5). The relevant fractions were evaporated under reduced pressure and stirred in ether. The solid was collected by filtration affording 280 mg (100% yield) of a white foam.

¹H NMR (DMSO_{d6}; δ ppm): 1.06-1.11 (2H, m); 1.17-1.27 (5H, m); 1.34 (9H, s); 1.67-1.76 (2H, d, J = 12); 1.86-1.99 (2H, m); 3.18-3.25 (2H, m); 3.40-3.50 (2H, m); 3.60-3.72 (1H, m); 3.77-3.85 (1H, m,); 3.89 (2H, s); 3.96-4.04 (1H, t, J = 7.3); 4.10 (1H, t, J = 9); 4.2-43 (1H, dd, J = 4.7 and J = 13.2); 4.38-4.44 (1H, d, J = 11.7); 4.83-4.95 (1H, m); 5.26 (2H, s); 7.20-7.55 (9H, m); 7.75 (1H,d, J = 12.6); 8.46 (1H, s).

MS (ESI): 847.5.

### 4. iii. 7-(4-{4-[(R)-5-((S)-2-tert-butoxycarbonylamino-propionyloxymethyl)-2-oxo-oxazolidin-3-yl]-2-fluoro-phenoxymethyl}-4-hydroxy-piperidin-1-yl)-1-cyclopropyl-6-fluoro-4-oxo-1,4-dihydro-quinoline-3-carboxylic acid:

A solution of intermediate 4.ii (285.3 mg) in dioxane/MeOH (1:1; 10 ml) was hydrogenated over 10% Pd/C (10 mg) for 4 h. The catalyst was removed by filtration and washed with MeOH (2 ml). The filtrate was evaporated under reduced pressure affording 215 mg (84.4% yield) of yellow foam.

MS (ESI): 757.3.

### 4.iv. 7-(4-{4-[(R)-5-((S)-2-amino-propionyloxymethyl)-2-oxo-oxazolidin-3 yl]-2-fluoro-phenoxymethyl}-4-hydroxy-piperidin-1-yl)-1-cyclopropyl-6-fluoro-4 oxo-1,4-dihydroquinoline-3-carboxylic acid hydrochloride:

A solution of intermediate 4.iii (193 mg) in dioxane (1 ml) was treated with 0.15 ml HCl (5*M* in dioxane). The reaction was stirred at rt for 14 h. The solvent was evaporated under reduced pressure. The residue was taken up in dioxane (10 ml) and the resulting solid was collected by filtration affording 153 mg (86.7% yield) of a yellow powder.

¹H NMR (DMSO_{d6}; δ ppm): 1.15-1.33 (4H, m); 1.35 (3H, d, J = 6.4); 1.73 (2H, d, J = 13); 1.93 (2H, m) ; 3.3 (2H, t, J = 11); 3.84 (4H, m); 4.13 (2H, m); 4.35 (1H, dd, J = 5 and J = 12); 4.55 (2H, dd, J = 2 and J = 12); 4.95 (1H, m); 7.23 (2H, m); 7.56 (2H, m); 7.88 (1H, d, J = 13.2); 8.51 (2H, sl); 8.64 (1H, s).

MS (ESI): 657.3.

### Example 5: 1-cyclopropyl-6-fluoro-7-{4-[2-fluoro-4-((R)-2-oxo-5-phosphonooxymethyl-oxazolidin-3-yl)-phenoxymethyl]-4-hydroxy-piperidin-1-yl}-4-oxo-1,4-dihydro-quinoline-3-carboxylic acid:

### 5.i. 7-(4-{4-[(R)-5-(bis-benzyloxy-phosphoryloxymethyl)-2-oxo-oxazolidin-3-yl]-2-fluoro-phenoxymethyl}-4-hydroxy-piperidin-1-yl)-1-cyclopropyl-6-fluoro-4-oxo-1,4-dihydro-quinoline-3-carboxylic acid:

A suspension of intermediate 1.iv (300 mg) and 4,5-dicyanoimidazole (109 mg) in DCM (3 ml) was treated at 0°C with dibenzyl *N,N*-diisopropylphosphoramidite (0.303 ml). The reaction was stirred at rt for 1 h. A 70% *tert*-butyl hydroperoxide solution in water (0.147 ml) was added. The solution was stirred 1 h at RT. The solvent was evaporated under reduced pressure and the residue was worked up (DCM) and purified by chromatography (DCM/MeOH 95:5), affording 214 mg (49.45 % yield) of an off-white foam.

MS (ESI): 846.3.

### 5.ii. 1-cyc/opropyl-6-fluoro-7-{4-[2-fluoro-4-((R)-1-oxo-5-phosphonooxymethyl-oxazolidin-3-yl)phenoxymethyl]-4-hydroxy-piperidin-1-yl}-4-oxo-1,4-dihydroquinoline-3-carboxylic acid:

A solution of intermediate 5.i (214 mg) in AcOH (1.5 ml) was treated with HBr (1.5 ml; 33% in AcOH). The reaction mixture was stirred at RT for 2 h and poured into water (20 ml). The gum was stirred for 1 h and decanted. The oily material was taken up in EA (20 ml) and further stirred for 2 h. The solid was collected by filtration and further stirred in DCM (10 ml). The solid was collected by filtration, affording 110 mg (65% yield) of a yellow powder.

¹H NMR (DMSO_{d6}; δ ppm): 1.18-1.30 (4H, m); 1.73 (1H,d, J = 13); 1.95 (2H,m); 2.40 (2H, d, J = 13); 3.26 (2H, m); 3.58 (2H, m); 3.76-3.87 (2H, m); 3.90 (1H,s); 3.95-4.15 (3H, m); 4.47 (1H, s); 4.86 (1H,m); 7.21 (2H, m); 7.57 (2H, m); 7.89 (1H, dd, J = 5.9 and J = 13.2); 8.65 (1H, s).

MS (ESI): 666.2.

### Example 6: 1-cyclopropyl-6-fluoro-7-{(RS)-3-[2-fluoro-4-((R)-5-hydroxymethyl-2-oxo-oxazolidin-3-yl)-phenoxymethyl]-3-hydroxy-pyrrolidin-1-yl}-4-oxo-1,4-dihydro-quinoline-3-carboxylic acid:

### 6.i. Diallyl-carbamic acid benzyl ester:

Benzoyl chloride (15.5 ml) was added dropwise over 30 min to a solution of diallylamine (12.3 ml) and TEA (21 ml) in DCM (100 ml) at 0 °C. The reaction mixture was stirred at rt for 16 h. The residue obtained after work up (DCM) was purified by chromatography (Hex/EA 95:5) to give 20.71 g (88% yield) of a colourless liquid.

¹H NMR (DMSO_{d6}; δ ppm): 3.83 (4H, dt, J = 1 and J = 6); 5.05-5.18 (6H, m); 5.70-5.86 (2H, m); 7.27-7.48 (5H, m).

### 6.ii. 2,5-dihydro-pyrrole-1-carboxylic acid benzyl ester:

Benzylidene-bis(tricyclohexylphosphine)dichlororuthenium (5 g) was added to a solution of intermediate 6.i (17.56 g) in DCM (1.5 1) at rt under nitrogen. The reaction mixture was stirred at 40 °C for 2 h and concentrated *in vacuo.* The residue was purified by chromatography (Hex/EA 90:10) to give 14.08 g (91% yield) of a yellow liquid.

¹H NMR (DMSO_{d6}; δ ppm): 4.05-4.16 (4H, m); 5.08 (2H, s); 5.81-5.92 (2H, m); 7.27-7.41 (5H,m).

### 6.iii. (RS)-3-hydroxy-pyrrolidine-1-carboxylic acid benzyl ester:

A 1*M* solution of borane in THF (9 ml) was added to a solution of intermediate 6.ii (1.81 g) in THF (25 ml) at 0 °C under nitrogen. The reaction mixture was stirred at rt for 16 h and was cooled to 0 °C. 20% NaOH (1.8 ml) was carefully added dropwise followed by 35% aq. hydrogen peroxide (1.2 ml). The mixture was stirred at 0 °C for 30 min and at rt for 2 h. Et₂O and an aq. 40% sodium bisulfite solution were added and the reaction mixture stirred vigorously for 15 min. The residue obtained after work up (Et₂O) was purified by chromatography (Hex/EA 5:5 to 3:7) to give 1.01 g (51% yield) of a colourless oil.

¹H NMR (DMSO_{d6}; δ ppm): 1.67-1.82 (1H, m); 1.82-1.96 (1H, m); 3.16-3.25 (1H, m); 3.28-3.44 (3H, m); 4.20-4.29 (1H, broad); 4.92 (1H, d, J = 3); 5.06 (2H, s); 7.27-7.41 (5H, m).

### 6.iv. 3-oxo-pyrrolidine-1-carboxylic acid benzyl ester:

A solution of intermediate 6.iii (1.10 g) in DCM (8 ml) was cooled to 0 °C and DIPEA (2.5 ml) was added dropwise, followed by a solution of sulfur trioxide pyridine complex (1.79 g) in DMSO (6.5 ml). The reaction mixture was stirred at 0 °C for 1 h and was quenched by the addition of water (6 ml). The aq. layer was extracted with Et₂O/Hex (1:1, 3 x 5 ml) and the combined org. layers were concentrated *in vacuo.* The residue obtained after work up (Et₂O/Hex 1:1) was purified by chromatography (Hex/EA 5:5) to give 1.05 g (96% yield) of a yellowish oil.

¹H NMR (DMSO_{d6}; δ ppm): 2.48-2.61 (2H, m); 3.61-3.80 (4H, m); 5.09 (2H, s); 7.27-7.41 (5H, m).

### 6.v. 3-methylene-pyrrolidine-1-carboxylic acid benzyl ester:

*t*-BuOK (617 mg) was added in one portion to a white suspension of methyl triphenylphosphonium bromide (1.98 g) in THF (10 ml) at rt under nitrogen. The yellow suspension was stirred at rt for I h and then cooled to -10 °C. A solution of intermediate 6.iv (1.05 g) in THF (2 ml) was added dropwise over 10 min and the reaction mixture was allowed to warm to rt over 2 h. The reaction was quenched by the addition of sat. aq. NH₄Cl (1 ml) and diluted with EA. The residue obtained after work up (EA) was purified by chromatography (Hex/EA 90:10) to give 633 mg (64% yield) of a yellowish liquid.

¹H NMR (DMSO_{d6}; δ ppm): 2.48-2.61 (2H, m); 3.36-3.53 (2H, m); 3.84-4.01 (2H, m); 4.97-5.03 (2H, m); 5.08 (2H, s); 7.27-7.41 (5H, m).

### 6.vi. 1-oxa-5-aza-spiro[2.4]heptane-5-carboxylic acid benzyl ester:

A solution of intermediate 6.v (7.21 g) in DCM (400 ml) was treated with MCPBA (20.1 g) and NaHCO₃ (22.3 g) at rt. The reaction was stirred at rt for 2 h, diluted with DCM (200 ml) and poured in a solution of Na₂SO₃ (45 g) in water (400 ml). The mixture was stirred for 10 min and the org. layer was separated. The residue obtained after work up (DCM) was purified by chromatography (Hex/EA 6:4) to give 4.37 g (56% yield) of a yellow oil.

¹H NMR (DMSO_{d6}; δ ppm): 1.70-1.83 (1H, m); 2.22-2.37 (1H, m); 2.90-2.94 (1H, m); 2.95-2.99 (1H, m); 3.15 (1H, t, J = 11); 3.39-3.77 (3H, m); 5.09 (2H, s); 7.27-7.41 (5H, m).

### 6.vii. (RS)-3-[2-fluoro-4-((R)-5-hydroxymethyl-1-oxo-oxazolidin-3-yl)-phenoxymethyl]-3-hydroxy-pyrrolidine-1-carboxylic acid benzyl ester:

K₂CO₃ (274 mg) was added to a suspension of intermediate 1.i (300 mg) and intermediate 6.vi (338 mg) in DMF (3 ml). The reaction mixture was stirred at 80 °C for 3 h and the solvent was removed *in vacuo.* The residue obtained after work up (DCM) was purified by chromatography (DCM/MeOH 95:5) to give 531 mg (87% yield) of a beige foam.

¹H NMR (DMSO_{d6}; δ ppm): 1.80-1.92 (1H, m); 1.96-2.08 (1H, m); 3.32-3.59 (5H, m); 3.66 (1H, ddd, J = 3, J = 6 and J = 13); 3.80 (1H, dd, J = 6 and J = 9); 3.97-4.09 (3H, m); 4.64-4.72 (1H, m); 5.07 (2H, s); 5.19 (1H, t, J = 6); 5.23 (1H, s); 7.18-7.23 (2H, m); 7.27-7.38 (5H, m); 7.57 (1H, dd, J = 2 and J = 14).

MS (ESI): 460.9.

### 6.viii. (R)-3-[3-fluoro-4-((RS)-3-hydroxy-pyrrolidin-3-ylmethoxy)-phenyl]-5-hydroxymethyl-oxazolidin-1-one:

A solution of intermediate 6.vii (259 mg) in THF/MeOH (1:1; 20 ml) was hydrogenated over 10% Pd/C (60 mg) for 20 h at rt. The reaction mixture was concentrated *in vacuo,* taken in DCM/MeOH 90:10 (20 ml) and stirred at rt for 30 min. The catalyst was filtered off and the filtrate concentrated *in vacuo* to give 184 mg (100% yield) of an orange foam.

MS (ESI): 327.3.

### 6.ix. 1-cyclopropyl-6-fluoro-7-{(RS)-3-[2-fluoro-4-((R)-5-hydroxymethyl-2-oxo-oxazolidin-3-yl)-phenoxymethyl]-3-hydroxy-pyrrolidin-1-yl}-4-oxo-1,4 dihydro-oxozolidin-3-yl)-phenoxymethyl]-3hydroxy-pyrrolidin-1yl}-4-oxo-1,4 dihydro-quinoline-3-carboxylic acid:

A solution of intermediate 6.viii (226 mg) and 7-chloro-1-cyclopropyl-6-fluoro-1,4-dihydro-4-oxo-3-quinolinecarboxylic acid boron diacetate complex (270 mg; prepared according to WO 88/07998) in NMP (5 ml) was treated with DIPEA (120 µl) and stirred at 60 °C for 2 h. The reaction mixture was concentrated *in vacuo* and the residue was taken in 5*M* HCl in MeOH (2 ml). The solution was stirred at rt for 1 h, concentrated *in vacuo* and the residue was purified by chromatography (DCM/MeOH/AcOH 95:4:1 to 90:9:1). The foamy residue was taken in MeOH (2 ml), stirred for 1 h and filtered. The crystals were collected and dried *in vacuo* to afford 23 mg (6% yield) of a beige solid.

¹H NMR (DMSO_{d6}; δ ppm): 1.10-1.34 (4H, m); 1.98-2.10 (1H, m); 2.14-2.26 (1H, m); 3.48-3.70 (3H, m); 3.71-3.89 (5H, m); 4.05 (1H, t, J = 9); 4.09-4.18 (2H, m); 4.66-4.74 (1H, m); 5.19 (1H, t, J = 6); 5.40 (1H, s); 7.09 (1H, d, J = 8); 7.18-7.31 (2H, m); 7.59 (1H, dd, J = 2 and J = 14); 7.82 (1H, d, J = 14); 8.59 (1H, s); 15.52 (1H, s).

MS (ESI): 572.3.

### Example 7: 1-cyclopropyl-6-fluoro-7-{(RS)-3-[2-fluoro-4-((R)-5-hydroxymethyl 2-oxo-oxazolidin-3-yl)-phenoxymethyl]-3-hydroxy-pyrrolidin-1-yl}-8-methoxy-4-oxo-1,4-dihydro-quinoline-3-carboxylic acid:

This compound was obtained as a yellow solid in 52% yield, starting from intermediate 6.viii (85 mg), 1-cyclopropyl-6,7-difluoro-1,4-dihydro-8-methoxy-4-oxo-3-quinolinecarboxylic acid boron diacetate complex (100 mg; prepared according to Sakurai et al., Bioorg. Med. Chem. Lett. (1998), 8, 2185-2190) and DIPEA (43 µl) and following the procedure of Example 6, step 6.ix. The foamy residue was stirred in EA (5 ml) and dried.

MS (ESI): 602.2.

### Example 8: 1-cyclopropyl-6-fluoro-7-{(RS)-3-[2-fluoro-4-((R)-5-hydroxymethyl-2-oxo-oxazolidin-3-yl)-phenoxymethyl]-pyrrolidin-1-yl}-4-oxo-1,4-dihydro-quinoline-3-carboxylic acid:

### 8.i. (RS)-3-hydroxymethyl-pyrrolidine-1-carboxylic acid benzyl ester:

This compound was obtained as a colourless oil in 68% yield, starting from intermediate 6.v (630 mg) and a 1***M*** solution of borane in THF (9 ml), and following the procedure of Example 6, step 6.iii.

¹H NMR (DMSO_{d6}; δ ppm): 1.51-1.71 (1H, m); 1.79-1.97 (1H, m); 2.19-2.35 (1H, m); 3.00-3.15 (1H, m); 3.19-3.50 (5H, m); 4.65 (1H, t, J = 5); 5.05 (2H, s); 7.27-7.40 (5H, m).

MS (ESI): 235.9.

### 8.ii. (RS)-3-{4-[(R)-5-(tert-butyl-dimethyl-silanyloxymethyl)-2-oxo-oxazolidin-3-yl]-2-fluoro-phenoxymethyl}-pyrrolidine-1-carboxylic acid benzyl ester:

A suspension of intermediate 8.i (456 mg), intermediate 2.ii (630 mg) and PPh₃ (726 mg) in THF (8 ml) was treated dropwise over 2 h with DIAD (0.55 ml) at rt. The reaction mixture was further stirred at rt for 2 h and was concentrated *in vacuo.* The residue was purified by chromatography (Hex/EA 7:3 to 6:4) to give 966 mg (94% yield) of a yellow oil.

¹H NMR (DMSO_{d6}; δ ppm): 0.04 (6H, s); 0.79 (9H, s); 1.65-1.82 (1H, m); 1.93-2.08 (1H, m); 2.56-2.74 (1H, m); 3.14-3.25 (1H, m); 3.30-3.59 (3H, m); 3.69-3.78 (1H, m); 3.74 (1H, dd, J = 3 and J = 12); 3.87 (1H, dd, J = 3 and J = 12); 3.95-4.12 (3H, m); 4.70-4.83 (1H, m); 5.05-5.08 (2H, m); 7.16-7.21 (2H, m); 7.27-7.38 (5H, m); 7.56 (1H, dd, J = 2 and J = 14).

MS (ESI): 559.3.

### 8.iii. (RS)-3-[2-fluoro-4-((R)-5-hydroxymethyl-2-oxo-oxazolidin-3-yl)-phenoxymethyl]-pyrrolidine-1-carboxylic acid benzyl ester:

A solution of intermediate 8.ii (200 mg) in dioxane (1 ml) was treated with 6*M* HCl in dioxane (0.30 ml) at rt. The reaction mixture was stirred at rt for 2 h and concentrated to dryness. The residue was diluted in DCM, washed with sat. aq. NaHCO₃ and worked up (DCM). The crude product was purified by FC (DCM/MeOH 98:2 to 96:4) to give 258 mg (81% yield) of a yellowish oil.

¹H NMR (DMSO_{d6}; δ ppm): 1.66-1.82 (1H, m); 1.96-2.10 (1H, m); 2.58-2.74 (1H, m); 3.14-3.26 (1H, m); 3.28-3.38 (1H, m); 3.39-3.61 (3H, m); 3.66 (1H, ddd, J = 4, J = 6 and J = 13); 3.80 (1H, dd, J = 6 and J = 9); 3.94-4.08 (3H, m); 4.62-4.71 (1H, m); 5.05 (2H, s); 5.19 (1H, t, J = 6); 7.18-7.21 (2H, m); 7.27-7.38 (5H, m); 7.57 (1H, dd, J = 2 and J = 14).

MS (ESI): 445.2.

### 8.iv. (R)-3-[3-fluoro-4-((RS)-1-pyrrolidin-3-ylmethoxy)-phenyl]-5-hydroxymethyl-oxazolidin-2-one:

This compound was prepared in 100% yield as a pinkish solid by hydrogenation of intermediate 8.iii (230 mg) over 10% Pd/C (72 mg) following the procedure of Example 6, step 6.viii.

MS (ESI): 311.3.

### 8.v. 1-cyclopropyl-6-fluoro-7-{(RS)-3-[2-fluoro-4-((R)-5-hydroxymethyl-2-oxo-oxazolidin-3-yl)-phenoxymethyl]-pyrrolidin-1-yl}-4-oxo-1,4-dihydro-quinoline-3-carboxylic acid:

This compound was prepared in 17% yield as a yellow solid, starting from intermediate 8.iv (67 mg), 7-chloro-1-cyclopropyl-6-fluoro-1,4-dihydro-4-oxo-3-quinolinecarboxylic acid boron diacetate complex (80 mg; prepared according to WO 88/07998) and DIPEA (36 µl), and following the procedure of Example 6, step 6.ix. The crude product was purified by chromatography (DCM/MeOH/AcOH 98:1:1 to 94:5:1) and the foamy residue was stirred in MeOH (1 ml).

¹H NMR (DMSO_{d6}; δ ppm): 1.10-1.34 (4H, m); 1.85-2.01 (1H, m); 2.14-2.28 (1H, m); 2.75-2.90 (1H, m); 3.48-3.60 (2H, m); 3.60-3.88 (6H, m); 4.05 (1H, t, J = 9); 4.09-4.18 (2H, m); 4.63-4.72 (1H, m); 5.19 (1H, t, J = 6); 7.09 (1H, d, J = 8); 7.18-7.29 (2H, m); 7.58 (1H, dd, J = 3 and J = 14); 7.80 (1H, d, J = 14); 8.58 (1H, s); 14.48 (1H, s).

MS (ESI): 556.3.

### Example 9: 1-cyclopropyl-6-fluoro-7-{(RS)-3-[2-fluoro-4-((R)-5-hydroxymethyl-2-oxo-oxazolidin-3-yl)-phenoxymethyl]-pyrrolidin-1-yl}-8-methoxy-4-oxo-1,4-dihydro-quinoline-3-carboxylic acid:

This compound was obtained as a yellow solid in 12% yield, starting from intermediate 8.iv (65 mg), 1-cyclopropyl-6,7-difluoro-1,4-dihydro-8-methoxy-4-oxo-3-quinolinecarboxylic acid boron diacetate complex (80 mg; prepared according to Sakurai et al., Bioorg. Med. Chem. Lett. (1998), 8, 2185-2190) and DIPEA (27 µl) and following the procedure of Example 6, step 6.ix. The crude product was purified by chromatography (DCM/MeOH/AcOH 98:1:1 to 94:5:1) and the foamy residue was stirred in EA/Et₂O (2:1; 1.5 ml).

¹H NMR (DMSO_{d6}; δ ppm): 0.90-1.18 (4H, m); 1.76-1.93 (1H, m); 2.10-2.24 (1H, m); 2.70-2.81 (1H, m); 3.57 (3H, s); 3.56-3.83 (7H, m); 3.99-4.20 (4H, m); 4.62-4.73 (1H, m); 5.19 (1H, t, J = 6); 7.18-7.29 (2H, m); 7.58 (1H, dd, J = 3 and J = 14); 7.68 (1H, d, J = 14); 8.64 (1H, s); 15.13 (1H, s).

MS (ESI): 586.3.

### Example 10: 1-cyclopropyl-6-fluoro-7-{4-[2-fluoro-4-((R)-5-hydroxymethyl-2-oxo-oxazolidin-3-yl)-phenoxymethyl]-4-phosphonooxy-piperidin-1-yl}-4-oxo-1,4-dihydro-quinoline-3-carboxylic acid:

### 10.i. 7-(4-{4-[5-(tert-butyl-dimethyl-silanyloxymethyl)-3-oxo-oxazolidin-3-yl]-2-fluoro-phenoxymethyl}-4-hydroxy-piperidin-1-yl)-1-cyclopropyl-6-fluoro-4-oxo-1,4-dihydroquinoline-3-carboxylic acid benzyl ester:

Imidazole (67 mg) and TBDMSCl (162 mg) were added to a solution of intermediate 4.i (600 mg) in DMF (6 ml). The solution was stirred at rt for 6 h. The solvent was evaporated under reduced pressure and the residue was worked up (DCM) and purified by chromatography (DCM/MeOH; 95:5), affording 544 mg (75.6% yield) of a yellow solid.

¹H NMR (DMSO_{d6}; δ ppm): 0.03 (6H, s); 0.78 (9H, s); 1.10 (2H, m); 1.22 (2H, m); 1.70 (2H, m); 1.92 (2H, m); 3.2 (2H, m); 3.45 (2H, m); 3.70 (3H, m); 3.83-3.88 (1H, dd, J = 2.6 and J = 12); 3.89 (2H, s); 4.07 (1H, t, J = 9); 4.73 (1H, m); 4.82 (1H, s); 5.26 (2H, s); 7.20 (2H, m); 7.28-7.58 (7H, m); 7.75 (1H, d, J = 13.5); 8.46 (1H, s).

MS (ESI): 790.5.

### 10.ii. 7-(4-(bis-benzyloxy-phosphoryloxy)-4-{4-[(R)-5-(tert-butyl-dlimethyl-silanyloxymethyl)-3-oxo-oxazoliclin-3-yl]-2-fluoro-phenoxymethyl)-piperidin-1-yl)-1-cyclopropyl-6-fluoro-4-oxo-1,4-dihydro-quinoline-3-carboxylic acid benzyl ester:

A suspension of intermediate 10.i (523 mg) in DCM (4 ml) was sequentially treated with 4,5-dicyanoimidazole (140 mg) and dibenzyl *N,N-*diisopropylphosphoramidite (392 µl). After 1 h at rt, the reaction mixture was treated with 190 µl *tert*-butyl hydroperoxide (70% in water) and further stirred at rt for 1 h. The solvent was evaporated under reduced pressure and the residue was worked up (DCM) and purified by chromatography (DCM/MeOH; 97.5:2.5). The resulting solid (369 mg) was stirred in ether (5 ml), affording 287 mg (41% yield) of a yellow solid.

¹H NMR (DMSO_{d6}; δ ppm): 0.02 (6H, s); 0.77 (9H, m); 1.07 (2H, m); 1.19 (2H, dd, J = 1.2 and J = 6.4), 2.07 (2H, t broad, J = 9); 2.29 (2H, d, J = 13.5); 3.18 (2H, t, J = 11); 3.49 (2H, m); 3.61 (1H, m); 3.73 (2H, m); 3.85 (1H, dd, J = 2.6 and J = 12); 4.07 (1H, t, J = 9.1); 4.38 (2H, s); 4.73 (1H, m); 5.02 (4H, d, J = 7.6); 5.26 (2H, s); 7.17 (2H, m); 7.42 (17H, m); 7.77 (1H, d, J = 13.2); 8.46 (1H, s).

MS (ESI): 1050.3.

### IO.iii. 7-{4-[4-((R)-S-acetotymethyl-2-oxo-oxazolidin-3-yl)-3-fluoro-phenoxymethyl]-4-phosphonooxy-piperidin-1-yl}-1-cyclopropyl-6-fluoro-4-oxo-1,4-dihydro-quinoline-3-carboxylic acid benzyl ester:

A suspension of intermediate 10.ii (200 mg) in AcOH (1.5 ml) was treated with HBr (1 ml; 33% in AcOH) and stirred at rt for 2 h. The reaction mixture was poured into cold water (10 ml). After work-up (DCM), the residue was stirred in ether (30 ml).

The resulting yellow solid was collected by filtration (144.5 mg; 95% yield).

MS (ESI): 798.1.

### 10.iv. 1-cyclopnopyl-6-fluoro-7-{4-[2-fluoro-4-((R)-5-hydromethyl-2-oxo-oxazolidin-3-yl)-phenoxymethyl]-4-phosphonooxy-piperidin-1-yl}-4-oro-1,4 dihydro-quiinoline-3-carboxylic acid:

A solution of intermediate 10.iii (144.5 mg) in dioxane/MeOH/water (3 ml; 1:1:1) was treated with sodium acetate (29 mg) and hydrogenated over Pd/C 10% (10 mag) for 10 h at rt. The catalyst was filtered off and washed with water (5 ml). The filtrate was evaporated under reduced pressure and taken up in water (1 ml) and treated with 1*N* HCl (400 µl) until precipitation. The solid was collected by filtration, redissolved in MeOH (10 ml) and treated with K₂CO₃ (56.25 mg). After stirring the mixture at rt for 30 min, the solvent was evaporated under reduced pressure and the residue was dissolved in water (4 ml) and treated with 1*N* HCl (814 µl) until precipitation. The solid was collected by filtration, washed with water and dried under reduced pressure, affording 105 mg (82% yield) of yellow solid.

¹H NMR (DMSOd₆; δ ppm): 1.13-1.30 (4H, m); 1.95-2.11 (2H, t broad, J = 10); 2.24 (2H, d, J = 12.5); 3.59 (3H, t, J = 10); 3.5-3.69 (3H, m); 3.74-3.87 (2H, m); 4.02 (1H, t, J = 9); 4.31 (2H, m); 4.66 (1H, m); 5.17 (1H, m); 7.19 (2H, m); 7.56 (2H, m); 7.87 (1H, d, J = 13.2); 8.63 (1H, s).

MS (ESI): 666.2.

### Example 11: 1-ethyl-6-fluoro-7-{4-[2-fluoro-4-((R)-5-hydroxymethyl-2-oxo-oxazolidin-3-yl)phenoxymethyl]-piperidin-1-yl)-4-oxo-1,4-dihydro-quinoline-3-carboxylic acid:

This compound was prepared in analogy to Example 1, step 1.iv, starting from intermediate 2.iv (250 mg) and 7-chloro-1-ethyl-6-fluoro-1,4-dihydro-4-oxo-3-quinolinecarboxylic acid boron complex (280 mg; prepared according to WO 87/03595). The residue obtained after evaporation of the solvent under reduced pressure was dissolved in water (100 ml) and DCM/MeOH (500 met; 9:1). The org. phase was dried over MgSO₄ and filtered. The filtrate was evaporated and stirred in DCM/MeOH (10 ml; 9:1). The yellow solid was collected by filtration (171 mg; 44% yield).

¹H NMR (DMSO_{d6}; δ ppm): 1.45 (3H, t, J = 7); 1.57 (2H, dq, J = 4.7 and J = 9); 1.95 (2H, d, J = 12.5); 2.01-2.09 (1H, m); 3.00 (2H, m); 3.62. (3H, dq, J = 4 and J = 13.2), 3.73-3.78 (1H, m); 3.80 (1H, dd, J = 6.5 and J = 9); 3.99-4.07 (3H, m); 4.49 (2H, q, J = 7), 4.65 (1H, m); 7.16 (3H, m); 7.47-7.54 (1H, m); 7.88 (1H, d, J = 13.8); 8.83 (1H, s).

MS (ESI): 558.2.

### Example 12: 7-(4-{4-[(R)-5-((S)-2-amino-propionyloxymethyl)-2-oxo-oxazolidin-3-yl]-2-fluoro-phenoxymethyl}-piperidin-1-yl)-1-cyclopropyl-6-fluoro-4-oxo-1,4-dihydro-quinoline-3-carboxylic acid hydrochloride:

### 12.i. 1-cyclopropyl-6-fluoro-7-{4-[2-fluoro-4-((R)-5-hydroxymethyl-2-oxo-oxazolidin-3-yl)phenoxymethyl]-piperidin-1-yl}-4-oxo-1,4-dihydro-quinoline-3-carboxylic acid benzyl ester:

A solution of the compound of Example 2 (600 mg) in DMF (10 ml) was treated with K₂CO₃ (155 mg) and BnBr (160 µl) and stirred at 80°C for 24 h. The solvent was evaporated under reduced pressure and the residue was worked up (DCM). The residue was stirred in ether (100 ml) and the resulting crystals were collected by filtration affording 612 mag (90.6% yield) of an off-white solid.

¹H NMR (DMSO_{d6}; δ ppm): 1.08-1.27 (4H, m); 1.45-1.60 (2H,m); 1.88-2.05 (3H, m); 2.89 (2H, t, J = 10.8); 3.50-3.57 (1H, m); 3.62-3.71 (4H, m); 3.78 (1H, dd, J = 6.2 and J = 8.8); 3.98 (2H, d, J = 6.2); 4.03 (1H, t, J = 8.8); 4.62-4.71 (1H, m); 5.17 (1H, t, J = 5.9); 5.25 (2H, s); 7.19-7.22 (2H, m); 7.31-7.41 (3H, m); 7.43-7.49 (3H, m); 7.53-7.55 (1H,dd, J = 2.0 and J=13.8); 7.75(1H,d, J = 13.8); 8.46 (1H,s).

MS (ESI): 660.3.

### 12.ii. 7-(4-{4-[(R)-5-((S)-2-tert-butoxycarbonylamino-propionyloxymethyl)-2-oxo-oxazolidin-3-yl]-2-fluoro-phenomethyl}-piperidin-1-yl)-1-cyclopropyl-6-fluoro-4-oxo-1,4-dihydro-quinoline-3-carboxylic acid benzyl ester:

This compound (307 mg; 81% yield) was obtained as a colourless solid in analogy to Example 4, step 4.ii, starting from intermediate 12.i (300 mg), Boc-L-Ala-OH (111 mg), EDC (113 mg) and DMAP (27 mg).

¹H NMR (DMSO_{d6}; δ ppm): 1.05-1.11 (2H, m);, 1.17-1.26 (5H, m); 1.32 (9H, s); 1.52 (2H, m); 1.96 (3H, m); 2.90 (2H, t, J = 11); 3.59-3.72 (3H, m); 3.81 (1H, dd, J = 6.7 and J = 9.7); 3.94-4.00 (3H, m); 4.12 (1H, t, J = 9); 4.25 (1H, dd, J = 4.7 and J = 11); 4.40 (1H, dd, J = 2.6 and J = 12.3); 4.85-4.95 (1H, m); 5.26 (2H, s); 718-7.23 (2H, m); 7.26-7.46 (7H, m); 7.75 (1H, d, J = 13.5); 8.45 (1H, s).

MS (ESI): 831.3.

### 12.iii. 7-(4-{4-((R)-5-((S)-2-tert-butoxycarbonylamino-propionyloxymethyl)-3-oxo-oxazolidin-3-yl]-2-fluoro-phenoxymethy}l-piperidin-1-yl)-1-cyclopropyl-6-fluoro-4-oxo-1,4-dihydro-quinoline-3-carboxylic acid:

This compound (252 mg; 100% yield) was obtained as a yellow solid in analogy to Example 4, step 4.iii by hydrogenation of intermediate 12.ii (279 mg) over Pd/C (10 mg).

¹H NMR(DMSO_{d6}; δ ppm): 1.12-1.22 (5H, m); 1.26-1.36 (11H, m); 1.47-1.59 (2H, m); 1.92 (2H, d, J = 12); 1.98-2.10 (1H, m); 2.99 (2H, t, J = 12); 3.79 (4H, m); 3.97 (3H, d, J = 7.6); 4.10 (1H, t, J = 9); 4.26 (1H, dd, J = 5.3 and J = 12.6); 4.36-4.43 (1H, m); 4.84-4.96 (1H, m); 7.17-7.32 (3H, m); 7.52 (1H, d, J = 12.9); 7.54 (1H, d, J = 8.2); 7.88 (1H, d, J = 13.5); 8.64 (1H, s).

MS (ESI): 741.3.

### 12.iv. 7-(4-{4-[(R)-5-((S)-2-amino-propionyloxymethyl)-1-oxo-oxazolidin-3yl]-2-fluoro-phenoxymethyl}-piperidin-1-yl)-1-cyclopropyl-6-fluoro-4-oxo-1,4 dihydroquinoline-3-carboxylic acid hydrochloride:

This compound (84 mg; 97% yield) was obtained as a yellow solid in analogy to Example 4, step 4.iv, starting from intermediate 12.iii (100 mg) and 5*M* HCl in dioxane (0.5 ml).

¹H NMR (DMSO_{d6}; δ ppm): 1.13-1.32 (4H, m); 1.36 (3H, d, J = 7.3); 1.46-1.61 (2H, m); 1.92(2H, d broad, J = 12); 1.98-2.10 (1H, m); 2.99 (2H, t, J = 12); 3.74-3.91 (5H, m); 3.98 (2H, d, J = 6.4); 4.10-4.18 (1H, m); 4.3 5 (1H, dd, J = 5.3 and J = 12.3); 4.55 (1H, dd, J = 2.6 and J = 12.3); 4.96 (1H, m); 7.19-7.24 (2H, m); 7.52-7.58 (2H, m); 7.88 (1H, d, J = 13.5); 8.47 (3H, m); 8.64 (1H, s).

MS (ESI): 641.2.

### Example 13: 6-fluoro-7-(4-[2-fluoro-4-((R)-5=hydroxymethyl-2-oxo-oxazolidin-3-yl)-phenoxymethyl]-4-hydroxy-piperidin-1-yl}-4-oxo-1,4-dihydro-quinoline-3-carboxylic acid:

A solution of intermediate 1.iv (150 mg) in DMA (10 ml) was hydrogenated over 20% Pd(OH)₂/C (100 mg) for 5 days at 80 °C. The reaction mixture was concentrated *in vacuo,* taken in DCM/MeOH 90:10 (50 ml) and stirred at rt for 30 min. The catalyst was filtered off and the filtrate concentrated *in vacuo.* The residue was taken in MeOH (0.2 ml) and EA (2 ml) was added. The suspension was stirred at rt for 30 min and filtered. The crystals were collected and dried *in vacuo* to give 48 mg (34% yield) of a yellow solid.

¹H NMR (DMSO_{d6}; δ ppm): 1.64-1.77 (2H, m); 1.82-1.98 (2H, m); 3.14-3.30 (2H, m); 3.38-3.59 (3H, m); 3.59-3.71 (1H, m); 3.78 (1H, dd, J = 6 and J = 9); 3.89 (2H, s); 4.03 (1H, t, J = 9); 4.62-4.73 (1H, m); 4.84 (1H, s); 5.17 (1H, t, J = 6); 7.16-7.23 (2H, m); 7.28 (1H, d, J = 8); 7.51-7.60 (1H, m); 7.81 (1H, d, J = 13); 8.79 (1H, s); 13.09 (1H, broad); 15.44 (1H, s).

MS (ESI): 546.2.

### Example 14: 6-fluoro-7-14-12-fluoro4-((R)-5-hydroxymethyl-2-oxo-oxazolidin-3-yl)-phenoxymethyl]-4-hydroxy-piperidin-1-yl}-4-oxo-1,4-dihydro-[1,8]naphthyridine-3-carboxylic acid:

A solution of intermediate 3.i (110 mg) in DMA (10 ml) was hydrogenated over 10% Pd/C (35 mg) for 16 h at 80 °C. The reaction mixture was concentrated *in vacuo,* taken in DCM/MeOH 90:10 (25 ml) and stirred at rt for 30 min. The catalyst was filtered off and the filtrate concentrated *in vacuo.* The residue was taken up in EA (2 ml), stirred at rt for 16 h and filtered. The crystals were collected and dried *in vacuo* to give 35 mg (34% yield) of a yellow solid.

¹H NMR (DMSO_{d6}; δ ppm): 1.62-1.92 (4H, m); 3.41-3.60 (3H, m); 3.60-3.69 (1H, m); 3.78 (1H, dd, J = 6 and J = 9); 3.86 (2H, s); 4.02 (1H, t, J = 8); 4.22-4.37 (2H, m); 4.60-4.72 (1H, m); 4.95 (1H, broad); 5.18 (1H, broad); 7.13-7.23 (2H, m); 7.50-7.59 (1H, m); 8.01 (1H, d, J = 14); 8.79 (1H, broad); 13.26 (1H, broad); 15.34 (1H, broad).

MS (ESI): 547.3.

### BIOLOGICAL ASSAYS

### ln vitro assay

### Experimental methods:

These assays have been performed following the description given in "Methods for dilution Antimicrobial Susceptibility Tests for Bacteria that Grow Aerobically, 4th ed.; Approved standard: NCCLS Document M7-A4; National Committee for Clinical Laboratory Standards: Villanova, PA, USA, 1997". Minimal inhibitory concentrations (MICs; mg/l) were determined in cation-adjusted Mueller-Hinton Broth (BBL) by a microdilution method following NCCLS guidelines (National Committee for Clinical Laboratory Standards. Methods for Dilution Antimicrobial Susceptibility). The pH of the test medium was 7.2-7.3.

In the particular case wherein the compound to be tested is a compound of formula I_{PDG} wherein at least one of R¹ and R² represents OPO₃H₂, then the test was carried out in the presence of human alkaline phosphatase (concentration: 1 U/ml).

In the particular case wherein the compound to be tested is a compound of formula I_{PDG} wherein R¹ represents a group OCOR⁵, then the test may be carried out in the presence of 50% human serum. However, this was not needed for the compounds of Examples 4 and 12 that already showed significant activity in the absence of human serum.

### Results:

All the above Examples were tested against several Gram positive and Gram negative bacteria. Typical antibacterial spectra are given in the table below (MIC in mg/l).

| **Example No.** | ***S. aureus* A798** | ***S. Pneumoniae* 49619** | ***M. catarrhalis* A894** |
|---|---|---|---|
| 3 | 0.25 | 0.125 | 0.5 |
| 7 | 1 | 0.25 | 0.063 |

Besides, the following results have been obtained for the Example compounds corresponding to formula I_{D} on *S. aureus* A798 (MIC in mg/l):

| **Example No.** | ***S. aureus* A798** | **Example No.** | ***S aureus* A798** |
|---|---|---|---|
| 1 | 0.25 | 8 | 0.125 |
| 2 | 0.25 | 9 | 1 |
| 3 | 0.25 | 11 | >16 |
| 6 | 1 | 13 | 2 |
| 7 | 1 | 14 | 4 |

In addition, the compound of Example 11 has a MIC of 8 mg/l against *E. faecalis* 29212 bacteria.

Moreover, in physiological environment (comprising phosphatases and esterases), the compounds of formula I_{PDG} are rapidly converted into the corresponding compounds of formula I_{D}. Indeed:
- the compounds of Examples 5 and 10, in the presence of human alkaline phosphatase, have MICs of respectively 0.25 and 0.5 mg/l against *S*. *aureus* A798, whereas the same compounds have MICs of respectively > 16 mg/l and 16 mg/l against *S*. *aureus* A798 when the phosphatase is absent; and
- the compounds of Examples 4 and 12, even in the absence of human serum, each have a MIC of 0.5 mg/l against *S*. *aureus* A798.

## Claims

1. A compound of the formula I 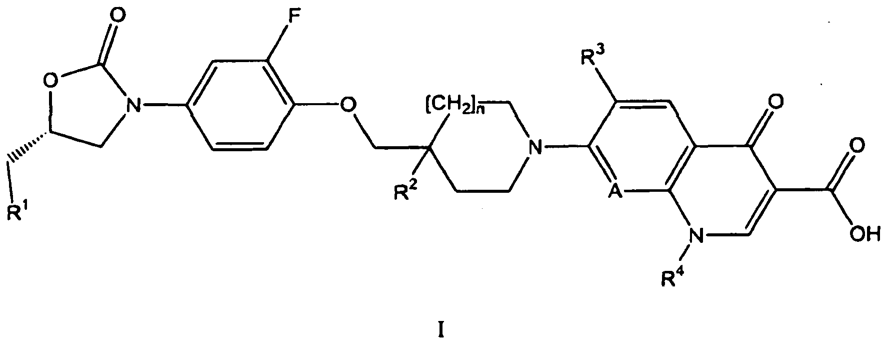 wherein
R¹ represents OH, OPO₃H₂ or OCOR⁵;
R² represents H, OH or OPO₃H₂;
A represents N or CR⁶;
R³ represents H or fluorine;
R⁴ is H, (C₁-C₃) alkyl or cycloalkyl;
R⁵ is the residue of a naturally occurring amino acid, of the enantiomer of a naturally occurring amino acid or of dimethylaminoglycine;
R⁶ represents H, alkoxy or halogen; and
n is 0 or 1;
or a salt of such a compound.

2. A compound of formula I according to claim 1, which is also a compound of formula I_{CE} 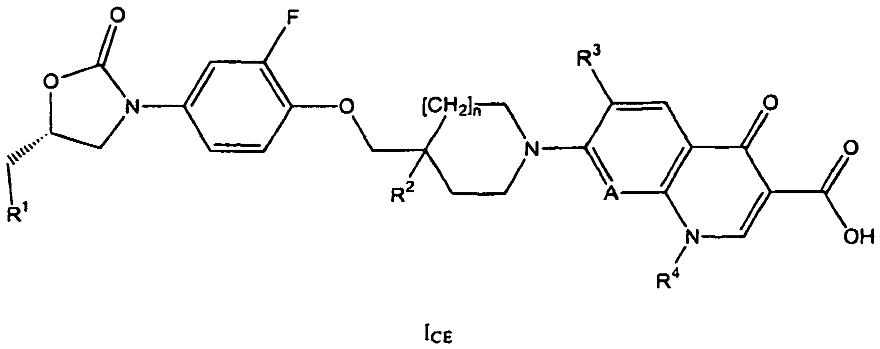 wherein
R¹ represents OH, OPO₃H₂ or OCOR⁵;
R² represents H, OH or OPO₃H₂;
A represents N or CR⁶;
R³ represents fluorine;
R⁴ represents H, (C₁-C₃) alkyl or cycloalkyl;
R⁵ is the residue of a naturally occurring amino acid (in particular the residue of Ala);
R⁶ represents H or alkoxy; and
n is 0 or 1;
or a salt of such a compound.

3. A compound of formula I according to claim 1, which is also a compound of formula I_{D} 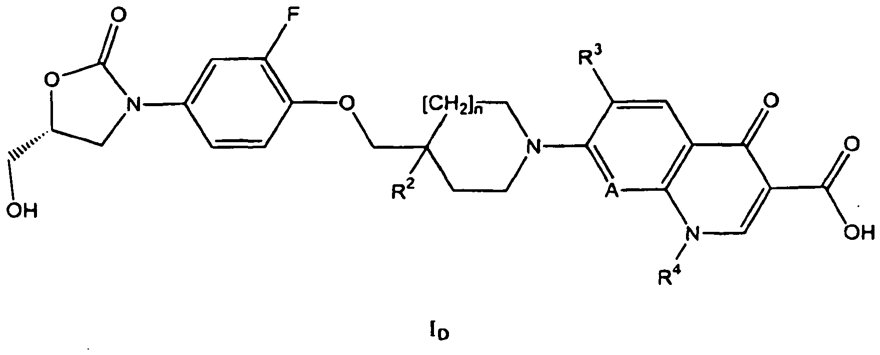 wherein
R² represents H or OH;
A represents N or CR⁶;
R³ represents fluorine;
R⁴ represents H, (C₁-C₃) alkyl or cycloalkyl;
R⁶ represents H or alkoxy; and
n is 0 or 1;
or a salt of such a compound.

4. A compound of formula I according to one of claims 1 to 3, wherein R² represents OH;
or a salt of such a compound.

5. A compound of formula I according to one of claims 1 to 3, wherein n is 0;
or a salt of such a compound.

6. A compound of formula I according to one of claims 1 to 3, wherein n is 1;
or a salt of such a compound.

7. A compound of formula I according to one of claims 1 to 3, wherein A is CR⁶, R⁶ representing H or alkoxy;
or a salt of such a compound.

8. A compound of formula I according to claim 7, wherein A is CR⁶, R⁶ representing H or methoxy;
or a salt of such a compound.

9. A compound of formula I according to one of claims 1 to 3, wherein R³ is fluorine;
or a salt of such a compound.

10. A compound of formula I according to one of claims 1 to 3, wherein R⁴ is cycloalkyl;
or a salt of such a compound.

11. A compound according to claim 1, which is selected from the following:
- 1-cyclopropyl-6-fluoro-7-{4-[2-fluoro-4-((*R*)-5-hydroxymethyl-2-oxo-oxazolidin-3-yl)-phenoxymethyl]-4-hydroxy-piperidin-1-yl}-4-oxo-1,4-dihydro-quinoline-3-carboxylic acid;
- 1-cyclopropyl-6-fluoro-7-{4-[2-fluoro-4-((*R*)-5-hydroxymethyl-2-oxo-oxazolidin-3-yl)-phenoxymethyl]-piperidin-1-yl}-4-oxo-1,4-dihydro-quinoline-3-carboxylic acid;
- 1-cyclopropyl-6-fluoro-7-{4-[2-fluoro-4-((*R*)-5-hydroxymethyl-2-oxo-oxazolidin-3-yl)-phenoxymethyl]-4-hydroxy-piperidin-1-yl}-4-oxo-1,4-dihydro-[1,8]naphthyridine-3-carboxylic acid;
- 7-(4-{4-[(*R*)-5-((*S*)-2-amino-propionyloxymethyl)-2-oxo-oxazolidin-3-yl]-2-fluoro-phenoxymethyl}-4-hydroxy-piperidin-1-yl)-1-cyclopropyl-6-fluoro-4-oxo-1,4-dihydroquinoline-3-carboxylic acid;
- 1-cyclopropyl-6-fluoro-7-{4-[2-fluoro-4-((*R*)-2-oxo-5-phosphonooxymethyl-oxazolidin-3-yl)-phenoxymethyl]-4-hydroxy-piperidin-1-yl}-4-oxo-1,4-dihydroquinoline-3-carboxylic acid;
- 1-cyclopropyl-6-fluoro-7-{(*R*)-3-[2-fluoro-4-((*R*)-5-hydroxymethyl-2-oxo-oxazolidin-3-yl)-phenoxymethyl]-3-hydroxy-pyrrolidin-1-yl}-4-oxo-1,4-dihydro-quinoline-3-carboxylic acid;
- 1-cyclopropyl-6-fluoro-7-{(*S*)-3-[2-fluoro-4-((*R*)-5-hydroxymethyl-2-oxo-oxazolidin-3-yl)-phenoxymethyl]-3-hydroxy-pyrrolidin-1-yl}-4-oxo-1,4-dihydro-quinoline-3-carboxylic acid;
- 1-cyclopropyl-6-fluoro-7-{(*R*)-3-[2-fluoro-4-((*R*)-5-hydroxymethyl-2-oxo-oxazolidin-3-yl)-phenoxymethyl]-3-hydroxy-pyrrolidin-1-yl}-8-methoxy-4-oxo-1,4-dihydroquinoline-3-carboxylic acid;
- 1-cyclopropyl-6-fluoro-7-{(*S*)-3-[2-fluoro-4-((*R*)-5-hydroxymethyl-2-oxo-oxazolidin-3-yl)-phenoxymethyl]-3-hydroxy-pyrrolidin-1-yl}-8-methoxy-4-oxo-1,4-dihydroquinoline-3-carboxylic acid;
- 1-cyclopropyl-6-fluoro-7-{(*R*)-3-[2-fluoro-4-((*R*)-5-hydroxymethyl-2-oxo-oxazolidin-3-yl)-phenoxymethyl]-pyrrolidin-1-yl}-4-oxo-1,4-dihydro-quinoline-3-carboxylic acid;
- 1-cyclopropyl-6-fluoro-7-{(*S*)-3-[2-fluoro-4-((*R*)-5-hydroxymethyl-2-oxo-oxazolidin-3-yl)-phenoxymethyl]-pyrrolidin-1-yl}-4-oxo-1,4-dihydro-quinoline-3-carboxylic acid;
- 1-cyclopropyl-6-fluoro-7-{(*R*)-3-[2-fluoro-4-((*R*)-5-hydroxymethyl-2-oxo-oxazolidin-3-yl)-phenoxymethyl]-pyrrolidin-1-yl}-8-methoxy-4-oxo-1,4-dihydro-quinoline-3-carboxylic acid;
- 1-cyclopropyl-6-fluoro-7-{(*S*)-3-[2-fluoro-4-((*R*)-5-hydroxymethyl-2-oxo-oxazolidin-3-yl)-phenoxymethyl]-pyrrolidin-1-yl}-8-methoxy-4-oxo-1,4-dihydro-quinoline-3-carboxylic acid;
- 1-cyclopropyl-6-fluoro-7-{4-[2-fluoro-4-((*R*)-5-hydroxymethyl-2-oxo-oxazolidin-3-yl)-phenoxymethyl]-4-phosphonooxy-piperidin-1-yl}-4-oxo-1,4-dihydro-quinoline-3-carboxylic acid;
- 1-ethyl-6-fluoro-7-{4-[2-fluoro-4-((*R*)-5-hydroxymethyl-2-oxo-oxazolidin-3-yl)-phenoxymethyl]-piperidin-l-yl}-4-oxo-1,4-dihydro-quinoline-3-carboxylic acid;
- 7-(4-{4-((*R*)-5-((*S*)-2-amino-propionyloxymethyl)-2-oxo-oxazolidin-3-yl]-2-fluoro-phenoxymethyl}-piperidin-1-yl)-1-cyclopropyl-6-fluoro-4-oxo-1,4-dihydro-quinoline-3-carboxylic acid;
- 6-fluoro-7-{4-[2-fluoro-4-((*R*)-5-hydroxymethyl-2-oxo-oxazolidin-3-yl)-phenoxymethyl]-4-hydroxy-piperidin-1-yl)-4-oxo-1,4-dihydro-quinoline-3-carboxylic acid;
- 6-fluoro-7-{4-[2-fluoro-4-((*R*)-5-hydroxymethyl-2-oxo-oxazolidin-3-yl)-phenoxymethyl]-4-hydroxy-piperidin-1-yl}-4-oxo-1,4-dihydro-[1,8] naphthyridine-3-carboxylic acid;
or a pharmaceutically acceptable salt of such a compound.

12. A compound according to claim 11, which is 1-cyclopropyl-6-fluoro-7-{4-[2-fluoro-4-((*R*)-5-hydroxymethyl-2-oxo-oxazolidin-3-yl)-phenoxymethyl]-4-hydroxy-piperidin-1-yl}-4-oxo-1,4-dihydro-quinoline-3-carboxylic acid;
or a pharmaceutically acceptable salt of such a compound.

13. As a medicament, a compound of formula I as defined in claim 1, or a pharmaceutically acceptable salt thereof.

14. A pharmaceutical composition containing, as active principle, a compound of formula I as defined in claim 1 or a pharmaceutically acceptable salt thereof, and at least one therapeutically inert excipient.

15. Use of a compound of formula I as defined in claim 1, or of a pharmaceutically acceptable salt thereof, for the manufacture of a medicament intended for the prevention or treatment of bacterial infections.

16. A compound of formula I as defined in claim 1, or a pharmaceutically acceptable salt thereof, for use in the prevention or treatment of bacterial infections.

## Patentansprüche

1. Verbindung der Formel 1 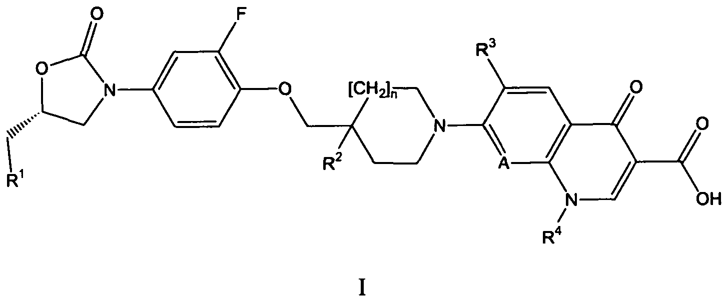 wobei
R¹ OH, OPO₃H₂ oder OCOR⁵ repräsentiert;
R² H, OH oder OPO₃H₂ repräsentiert;
A N oder CR⁶ repräsentiert;
R³ H oder Fluor repräsentiert;
R⁴ H, (C₁-C₃)Alkyl oder Cycloalkyl ist;
R⁵ der Rest einer natürlich vorkommenden Aminosäure, des Enantiomers einer natürlich vorkommenden Aminosäure oder von Dimethylaminoglycin ist;
R⁶ H, Alkoxy oder Halogen repräsentiert; und
n 0 oder 1 ist;
oder ein Salz einer solchen Verbindung.

2. Verbindung der Formel I nach Anspruch I, die auch eine Verbindung der Formel I_{CE} ist 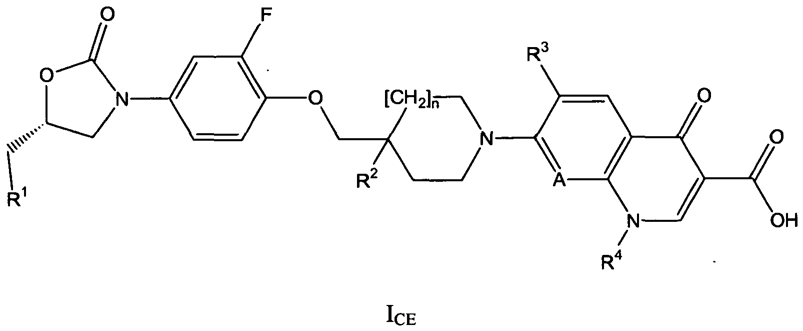 wobei
R¹ OH, OPO₃H₂ oder OCOR⁵ repräsentiert;
R² H, OH oder OPO₃H₂ repräsentiert;
A N oder CR⁶ repräsentiert;
R³ Fluor repräsentiert;
R⁴ H, (C₁-C₃)Alkyl oder Cycloalkyl repräsentiert;
R⁵ der Rest einer natürlich vorkommenden Aminosäure (insbesondere der Rest von Ala) ist;
R⁶ H oder Alkoxy repräsentiert; und
n 0 oder 1 ist;
oder ein Salz einer solchen Verbindung.

3. Verbindung der Formel I nach Anspruch 1, die auch eine Verbindung der Formel I_{D} ist 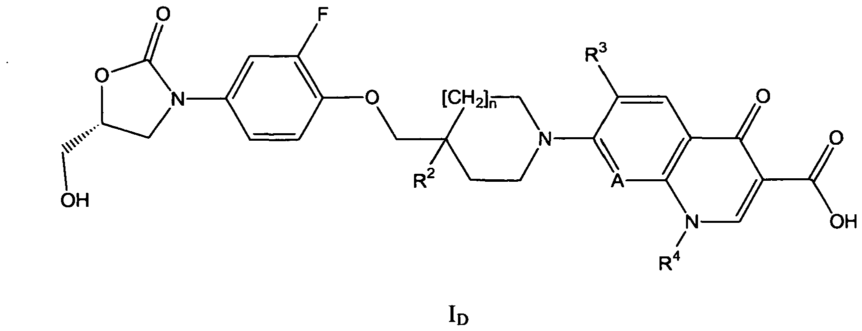 wobei
R² H oder OH repräsentiert;
A N oder CR⁶ repräsentiert;
R³ Fluor repräsentiert;
R⁴ H, (C₁-C₃)Alkyl oder Cycloalkyl repräsentiert;
R⁶ H oder Alkoxy repräsentiert; und
n 0 oder 1 ist;
oder ein Salz einer solchen Verbindung.

4. Verbindung der Formel I nach einem der Ansprüche 1 bis 3, wobei R² OH repräsentiert;
oder ein Salz einer solchen Verbindung.

5. Verbindung der Formel I nach einem der Ansprüche 1 bis 3, wobei n 0 ist;
oder ein Salz einer solchen Verbindung.

6. Verbindung der Formel I nach einem der Ansprüche 1 bis 3, wobei n 1 ist;
oder ein Salz einer solchen Verbindung.

7. Verbindung der Formel I nach einem der Ansprüche 1 bis 3, wobei A CR⁶ ist, wobei R⁶ H oder Alkoxy repräsentiert;
oder ein Salz einer solchen Verbindung.

8. Verbindung der Formel I nach Anspruch 7, wobei A CR⁶ ist, wobei R⁶ H oder Methoxy repräsentiert;
oder ein Salz einer solchen Verbindung.

9. Verbindung der Formel I nach einem der Ansprüche 1 bis 3, wobei R³ Fluor ist;
oder ein Salz einer solchen Verbindung.

10. Verbindung der Formel I nach einem der Ansprüche 1 bis 3, wobei R⁴ Cycloalkyl ist;
oder ein Salz einer solchen Verbindung.

11. Verbindung nach Anspruch 1, die ausgewählt ist aus den Folgenden:
- 1-Cyclopropyl-6-fluor-7-{4-[2-fluor-4-((*R*)-5-hydroxymethyl-2-oxo-oxazolidin-3-yl)-phenoxymethyl]-4-hydroxy-piperidin-1-yl}-4-oxo-1,4-dihydro-chinolin-3-carbonsäure;
- 1-Cyclopropyl-6-fluor-7-{4-[2-fluor-4-((*R*)-5-hydroxymethyl-2-oxo-oxazolidin-3-yl)-phenoxymethyl]-piperidin-1-yl}-4-oxo-1,4-dihydro-chinolin-3-carbonsäure;
- 1-Cyclopropyl-6-fluor-7-{4-[2-fluor-4-((*R*)-5-hydroxymethyl-2-oxo-oxazolidin-3-yl)-phenoxymethyl]-4-hydroxy-piperidin-1-yl}-4-oxo-1,4-dihydro-[1,8]naphthyridin-3-carbonsäure;
- 7-(4-{4-[(*R*)-5-((*S*)-2-Amino-propionyloxymethyl)-2-oxo-oxazolidin-3-yl]-2-fluor-phenoxymethyl}-4-hydroxy-piperidin-1-yl)-1-cyclopropyl-6-fluor-4-oxo-1,4-dihydro-chinolin-3-carbonsäure;
- 1-Cyclopropyl-6-fluor-7-{4-[2-fluor-4-((*R*)-2-oxo-5-phosphonooxymethyl-oxazolidin-3-yl)-phenoxymethyl]-4-hydroxy-piperidin-1-yl}-4-oxo-1,4-dihydro-chinolin-3-carbonsäure;
- 1-Cyclopropyl-6-fluor-7-{(*R*)-3-[2-fluor-4-((*R*)-5-hydroxymethyl-2-oxo-oxazolidin-3-yl)-phenoxymethyl]-3-hydroxy-pyrrolidin-1-yl}-4-oxo-1,4-dihydro-chinolin-3-carbonsäure;
- 1-Cyclopropyl-6-fluor-7-{(*S*)-3-[2-fluor-4-((*R*)-5-hydroxymethyl-2-oxo-oxazolidin-3-yl)-phenoxymethyl]-3-hydroxy-pyrrolidin-1-yl}-4-oxo-1,4-dihydro-chinolin-3-carbonsäure;
- 1-Cyclopropyl-6-fluor-7-{(*R*)-3-[2-fluor-4-((*R)*-5-hydroxymethyl-2-oxo-oxazolidin-3-yl)-phenoxymethyl]-3-hydroxy-pyrrolidin-1-yl}-8-methoxy-4-oxo-1,4-dihydro-chinolin-3-carbonsäure;
- 1-Cyclopropyl-6-fluor-7-{(*S*)-3-[2-fluor-4-((*R*)-5-hydroxymethyl-2-oxo-oxazolidin-3-yl)-phenoxymethyl]-3-hydroxy-pyrrolidin-1-yl}-8-methoxy-4-oxo-1,4-dihydro-chinolin-3-carbonsäure;
- 1-Cyclopropyl-6-fluor-7-{(*R*)-3-[2-fluor-4-((*R*)-5-hydroxymethyl-2-oxo-oxazolidin-3-yl)-phenoxymethyl]-pyrrolidin-1-yl}-4-oxo-1,4-dihydro-chinolin-3-carbonsäure;
- 1-Cyclopropyl-6-fluor-7-{(*S*)-3-[2-fluor-4-((*R*)-5-hydroxymethyl-2-oxo-oxazolidin-3-yl)-phenoxymethyl]-pyrrolidin-1-yl}-4-oxo-1,4-dihydro-chinolin-3-carbonsäure;
- 1-Cyclopropyl-6-fluor-7-{(*R*)-3-[2-fluor-4-((*R*)-5-hydroxymethyl-2-oxo-oxazolidin-3-yl)-phenoxymethyl]-pyrrolidin-1-yl}-8-methoxy-4-oxo-1,4-dihydro-chinolin-3-carbonsäure;
- 1-Cyclopropyl-6-fluor-7-{(*S*)-3-[2-fluor-4-((*R*)-5-hydroxymethyl-2-oxo-oxazolidin-3-yl)-phenoxymethyl]-pyrrolidin-1-yl}-8-methoxy-4-oxo-1,4-dihydro-chinolin-3-carbonsäure;
- 1-Cyclopropyl-6-fluor-7-{4-[2-fluor-4-((*R*)-5-hydroxymethyl-2-oxo-oxazolidin-3-yl)-phenoxymethyl]-4-phosphonooxy-piperidin-1-yl}-4-oxo-1,4-dihydro-chinolin-3-carbonsäure;
- 1-Ethyl-6-fluor-7-{4-[2-fluor-4-((*R*)-5-hydroxymethyl-2-oxo-oxazolidin-3-yl)-phenoxymethyl]-piperidin-1-yl}-4-oxo-1,4-dihydro-chinolin-3-carbonsäure;
- 7-(4-{4-[(*R*)-5-((*S*)-2-Amino-propionyloxymethyl)-2-oxo-oxazolidin-3-yl]-2- fluorphenoxymethyl}-piperidin-1-yl)-1-cyclopropyl-6-fluor-4-oxo-1,4-dihydro-chinolin-3-carbonsäure;
- 6-Fluor-7-{4-[2-fluor-4-((*R*)-5-hydroxymethyl-2-oxo-oxazolidin-3-yl)-phenoxymethyl]-4-hydroxy-piperidin-1-yl}-4-oxo-1,4-dihydro-chinolin-3-carbonsäure;
- 6-Fluor-7-{4-[2-fluor-4-((*R*)-5-hydroxymethyl-2-oxo-oxazolidin-3-yl)-phenoxymethyl]-4-hydroxy-piperidin-1-yl}-4-oxo-1,4-dihydro-[1,8]naphthyridin-3-carbonsäure;
oder ein pharmazeutisch akzeptables Salz einer solchen Verbindung.

12. Verbindung nach Anspruch 11, die 1-Cyclopropyl-6-fluor-7-{4-[2-fluor-4-(*(R*)-5-hydroxymethyl-2-oxo-oxazolidin-3-yl)-phenoxymethyl]-4-hydroxy-piperidin-1-yl}-4-oxo-1,4-dihydro-chinolin-3-carbonsäure ist;
oder ein pharmazeutisch akzeptables Salz einer solchen Verbindung.

13. Als Medikament, eine Verbindung der Formel I nach Anspruch 1 oder ein pharmazeutisch akzeptables Salz davon.

14. Pharmazeutische Zusammensetzung, die als Wirkstoff eine Verbindung der Formel I nach Anspruch 1 oder ein pharmazeutisch akzeptables Salz davon und wenigstens einen therapeutisch inerten Exzipienten enthält.

15. Verwendung einer Verbindung der Formel I nach Anspruch 1 oder eines pharmazeutisch akzeptablen Salzes davon zur Herstellung eines Medikaments zur Verhütung oder Behandlung von bakteriellen Infektionen.

16. Verbindung der Formel I nach Anspruch 1 oder ein pharmazeutisch akzeptables Salz davon zur Verwendung bei der Verhütung oder Behandlung von bakteriellen Infektionen.

## Revendications

1. Composé de formule 1 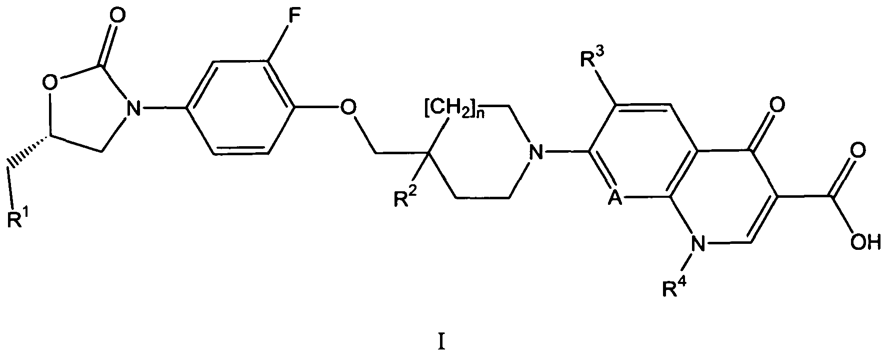 où
R¹ représente OH, OPO₃H₂ ou OCOR⁵ ;
R² représente H, OH ou OPO₃H₂ ;
A représente N ou CR⁶ ;
R³ représente H ou un fluor ;
R⁴ représente H ou un (C₁-C₃)alkyle ou cycloalkyle ;
R⁵ est le résidu d'un acide aminé existant naturellement, de l'énantiomère d'un acide aminé existant naturellement ou de la diméthylaminoglycine ;
R⁶ représente H, un alkoxy ou un halogène ; et
n représente 0 ou 1 ;
ou sel d'un tel composé.

2. Composé de formule 1 selon la revendication 1, qui est également un composé de formule I_{CE} 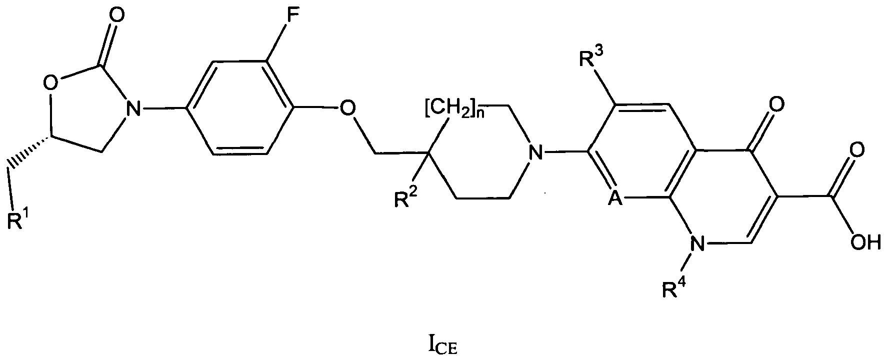 où
R¹ représente OH, OPO₃H₂ ou OCOR⁵
R² représente H, OH ou OPO₃H₂ ;
A représente N ou CR⁶ ;
R³ représente un fluor ;
R⁴ représente H ou un (C₁-C₃)alkyle ou cycloalkyle ;
R⁵ est le résidu d'un acide aminé existant naturellement (en particulier le résidu de l'Ala) ;
R⁶ représente H ou un alkoxy ; et
n représente 0 ou 1 ;
ou sel d'un tel composé.

3. Composé de formule 1 selon la revendication 1, qui est également un composé de formule I_{D} 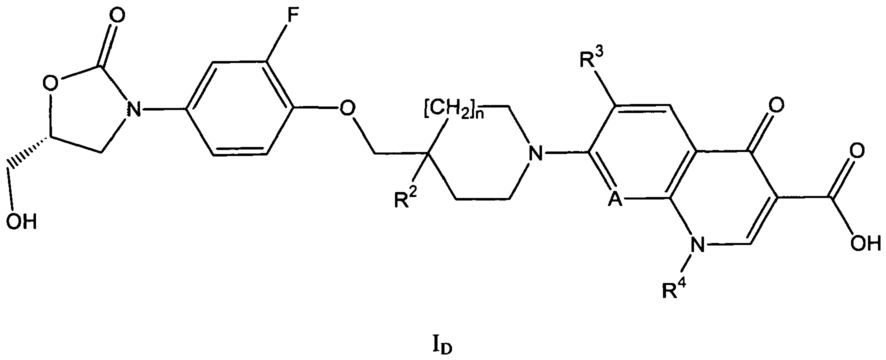 où
R² représente H ou OH ;
A représente N ou CR⁶ ;
R³ représente un fluor ;
R⁴ représente H ou un (C₁-C₃)alkyle ou cycloalkyle ;
R⁶ représente H ou un alkoxy ; et
n représente 0 ou 1 ;
ou sel d'un tel composé.

4. Composé de formule 1 selon l'une des revendications 1 à 3, où R² représente OH ;
ou sel d'un tel composé.

5. Composé de formule 1 selon l'une des revendications 1 à 3, où n représente 0 ;
ou sel d'un tel composé.

6. Composé de formule 1 selon l'une des revendications 1 à 3, où n représente 1 ;
ou sel d'un tel composé.

7. Composé de formule 1 selon l'une des revendications 1 à 3, où A représente CR⁶, R⁶ représentant H ou un alkoxy ;
ou sel d'un tel composé.

8. Composé de formule 1 selon la revendication 7, où A représente CR⁶ R⁶ représentant H ou un méthoxy ;
ou sel d'un tel composé.

9. Composé de formule 1 selon l'une des revendications 1 à 3, où R³ représente un fluor ;
ou sel d'un tel composé.

10. Composé de formule 1 selon l'une des revendications 1 à 3, où R⁴ représente un cycloalkyle ;
ou sel d'un tel composé.

11. Composé selon la revendication 1, qui est sélectionné parmi les suivants :
- Acide 1-cyclopropyl-6-fluoro-7-{4-[2-fluoro-4-((*R*)-5-hydroxyméthyl-2-oxo-oxazolidin-3-yl)-phénoxyméthyl]-4-hydroxy-pipéridin-1-yl}-4-oxo-1,4-dihydro-quinoléine-3-carboxylique ;
- Acide 1-cyclopropyl-6-fluoro-7-{4-[2-fluoro-4-((*R*)-5-hydroxyméthyl-2-oxo-oxazolidin-3-yl)-phénoxyméthyl]-pipéridin-1-yl}-4-oxo-1,4-dihydro-quinoléine-3-carboxylique ;
- Acide 1-cyclopropyl-6-fluoro-7-{4-[2-fluoro-4-((*R*)-5-hydroxyméthyl-2-oxo-oxazolidin-3-yl)-phénoxyméthyl]-4-hydroxy-pipéridin-1-yl}-4-oxo-1,4-dihydro-[1,8]naphtyridine-3-carboxylique ;
- Acide 7-(4-{4-[(*R*)-5-((*S*)-2-amino-propionyloxyméthyl)-2-oxo-oxazolidin-3-yl]-2-fluoro-phénoxyméthyl}-4-hydroxy-pipéridin-1-yl)-1-cyclopropyl-6-fluoro-4-oxo-1,4-dihydro-quinoléine-3-carboxylique ;
- Acide 1-cyclopropyl-6-fluoro-7-{4-[2-fluoro-4-((*R*)-2-oxo-5-phosphonooxyméthyl-oxazolidin-3-yl)-phénoxyméthyl]-4-hydroxy-pipéridin-1-yl}-4-oxo-1,4-dihydro-quinoléine-3-carboxylique ;
- Acide 1-cyclopropyl-6-fluoro-7-{(*R*)-3-[2-fluoro-4-((*R*)-5-hydroxyméthyl-2-oxo-oxazolidin-3-yl)-phénoxyméthyl]-3-hydroxy-pyrrolidin-1-yl}-4-oxo-1,4-dihydro-quinoléine-3-carboxylique ;
- Acide 1-cyclopropyl-6-fluoro-7-{(*S*)-3-[2-fluoro-4-((*R*)-5-hydroxyméthyl-2-oxo-oxazolidin-3-yl)-phénoxyméthyl]-3-hydroxy-pyrrolidin-1-yl}-4-oxo-1,4-dihydro-quinoléine-3-carboxylique ;
- Acide 1-cyclopropyl-6-fluoro-7-{(*R*)-3-[2-fluoro-4-((*R*)-5-hydroxyméthyl-2-oxo-oxazolidin-3-yl)-phénoxyméthyl]-3-hydroxy-pyrrolidin-1-yl}-8-méthoxy-4-oxo-1,4-dihydro-quinoléine-3-carboxylique ;
- Acide 1-cyclopropyl-6-fluoro-7-{(*S*)-3-[2-fluoro-4-((*R*)-5-hydroxyméthyl-2-oxo-oxazolidin-3-yl)-phénoxyméthyl]-3-hydroxy-pyrrolidin-1-yl}-8-méthoxy-4-oxo-1,4-dihydro-quinoléine-3-carboxylique ;
- Acide 1-cyclopropyl-6-fluoro-7-{(*R*)-3-[2-fluoro-4-((*R*)-5-hydroxyméthyl-2-oxo-oxazolidin-3-yl)-phénoxyméthyl]-pyrrolidin-1-yl}-4-oxo-1,4-dihydro-quinoléine-3-carboxylique ;
- Acide 1-cyclopropyl-6-fluoro-7-{(*S*)-3-[2-fluoro-4-((*R*)-5-hydroxyméthyl-2-oxo-oxazolidin-3-yl)-phénoxyméthyl]-pyrrolidin-1-yl}-4-oxo-1,4-dihydro-quinoléine-3-carboxylique ;
- Acide 1-cyclopropyl-6-fluoro-7-{(*R*)-3-[2-fluoro-4-((*R*)-5-hydroxyméthyl-2-oxo-oxazolidin-3-yl)-phénoxyméthyl]-pyrrolidin-1-yl}-8-méthoxy-4-oxo-1,4-dihydro-quinoléine-3-carboxylique ;
- Acide 1-cyclopropyl-6-fluoro-7-{(*S*)-3-[2-fluoro-4-((*R*)-5-hydroxyméthyl-2-oxo-oxazolidin-3-yl)-phénoxyméthyl]-pyrrolidin-1-yl}-8-méthoxy-4-oxo-1,4-dihydro-quinoléine-3-carboxylique ;
- Acide 1-cyclopropyl-6-fluoro-7-{4-[2-fluoro-4-((*R*)-5-hydroxyméthyl-2-oxo-oxazolidin-3-yl)-phénoxyméthyl]-4-phosphonooxy-pipéridin-1-yl}-4-oxo-1,4-dihydro-quinoléine-3-carboxylique ;
- Acide 1-éthyl-6-fluoro-7-{4-[2-fluoro-4-((*R*)-5-hydroxyméthyl-2-oxo-oxazolidin-3-yl)-phénoxyméthyl]-pipéridin-1-yl}-4-oxo-1,4-dihydro-quinoléine-3-carboxylique ;
- Acide 7-(4-{4-[(*R*)-5-((*S*)-2-amino-propionyloxyméthyl)-2-oxo-oxazolidin-3-yl]-2-fluoro-phénoxyméthyl}-pipéridin-1-yl)-1-cyclopropyl-6-fluoro-4-oxo-1,4-dihydro-quinoléine-3-carboxylique ;
- Acide 6-fluoro-7- {4-[2-fluoro-4-((*R*)-5-hydroxyméthyl-2-oxo-oxazolidin-3-yl)-phénoxyméthyl]-4-hydroxy-pipéridin-1-yl}-4-oxo-1,4-dihydro-quinoléine-3-carboxylique ;
- Acide 6-fluoro-7-{4-[2-fluoro-4-((*R*)-5-hydroxyméthyl-2-oxo-oxazolidin-3-yl)-phénoxyméthyl]-4-hydroxy-pipéridin-1-yl}-4-oxo-1,4-dihydro-[1,8]naphtyridine-3-carboxylique ;
ou sel pharmaceutiquement acceptable d'un tel composé.

12. Composé selon la revendication 11, qui est l'acide 1-cyclopropyl-6-fluoro-7-{4-[2-fluoro-4-((*R*)-5-hydroxyméthyl-2-oxo-oxazolidin-3-yl)-phénoxyméthyl]-4-hydroxy-pipéridin-1-yl}-4-oxo-1,4-dihydro-quinoléine-3-carboxylique.
ou sel pharmaceutiquement acceptable d'un tel composé.

13. En tant que médicament, composé de formule I tel que défini à la revendication 1 ou sel pharmaceutiquement acceptable de celui-ci.

14. Composition pharmaceutique contenant, comme principe actif, un composé de formule I tel que défini à la revendication 1 ou un sel pharmaceutiquement acceptable de celui-ci, et au moins un excipient thérapeutiquement inerte.

15. Utilisation d'un composé de formule I tel que défini à la revendication 1, ou d'un sel pharmaceutiquement acceptable de celui-ci, dans la fabrication d'un médicament indiqué dans la prévention ou le traitement d'infections bactériennes.

16. Composé de formule I tel que défini à la revendication 1. ou sel pharmaceutiquement acceptable de celui-ci, en vue d'une utilisation dans la prévention ou le traitement d'infections bactériennes.
